(19) Europäisches Patentamt
European Patent Office
Office européen des brevets

(11) **EP 2 982 683 B1**

(12) **EUROPEAN PATENT SPECIFICATION**

(45) Date of publication and mention
of the grant of the patent:
**14.08.2019 Bulletin 2019/33**

(51) Int Cl.:
*C07K 14/285* (2006.01)   *C07K 19/00* (2006.01)
*C12N 15/31* (2006.01)   *A61K 39/102* (2006.01)
*A61K 39/116* (2006.01)   *A61P 31/04* (2006.01)
*G01N 33/53* (2006.01)

(21) Application number: **14768194.4**

(22) Date of filing: **21.03.2014**

(86) International application number:
**PCT/CN2014/073851**

(87) International publication number:
**WO 2014/146603 (25.09.2014 Gazette 2014/39)**

(54) **RECOMBINANT PASTEURELLA MULTOCIDA TOXIN AND APPLICATION THEREOF**

REKOMBINANTES PASTEURELLA MULTOCIDA-TOXIN UND VERWENDUNG DAVON

PROTEINE RECOMBINANTE DE LA TOXINE DE PASTEURELLA MULTOCIDA ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.03.2013 CN 201310095061**

(43) Date of publication of application:
**10.02.2016 Bulletin 2016/06**

(73) Proprietor: **SBC Virbac Biotech Co. Ltd.
Taipei (TW)**

(72) Inventors:
• **YANG, Ying-Chen
Taipei City (TW)**
• **KUO, Tsun-Yung
I-Lan (TW)**

(74) Representative: **Wittmann, Günther
Patentanwaltskanzlei Wittmann
Frans-Hals-Straße 31
81479 München (DE)**

(56) References cited:
**CN-A- 1 736 479     CN-A- 101 152 570**

• **Lee ET AL: "Antigenicity of Partial Fragments of Recombinant Pasteurella multocida Toxin", J. Microbiol. Biotechnol, 1 January 2010 (2010-01-01), pages 1756-1763, XP055288816, DOI: 10.4014/jmb.1004.04036 Retrieved from the Internet: URL:http://www.jmb.or.kr/journal/viewJourn al.html?year=2010&vol=20&num=12&page=175 6 [retrieved on 2016-07-14]**
• **J. LEE ET AL: "Protective Immunity Conferred by the C-Terminal Fragment of Recombinant Pasteurella multocida Toxin", CLINICAL AND VACCINE IMMUNOLOGY, vol. 19, no. 9, 1 September 2012 (2012-09-01), pages 1526-1531, XP055288361, US ISSN: 1556-6811, DOI: 10.1128/CVI.00238-12**
• **RAVI V. KOLLA ET AL: "Complement C3d Conjugation to Anthrax Protective Antigen Promotes a Rapid, Sustained, and Protective Antibody Response", PLOS ONE, vol. 2, no. 10, 17 October 2007 (2007-10-17), page e1044, XP055288360, DOI: 10.1371/journal.pone.0001044**
• **GOR D O ET AL: "Genetic fusion of three tandem copies of murine C3d sequences to diphtheria toxin fragment B elicits a decreased fragment B-specific antibody response", IMMUNOLOGY LETTERS, ELSEVIER BV, NL, vol. 102, no. 1, 15 January 2006 (2006-01-15), pages 38-49, XP024999108, ISSN: 0165-2478, DOI: 10.1016/J.IMLET.2005.06.020 [retrieved on 2006-01-15]**

EP 2 982 683 B1

- **TANG, XIBIAO ET AL.: 'Characteristics and Immunogenicity of the N-terminal and C-terminal Recombinants of Pasteurella Multocida Toxin' ACTA MICROBIOLOGICA SINICA vol. 48, no. 2, 04 February 2008, pages 213 - 219, XP055287038**
- **SEO J. ET AL.: 'Expression of 4 Truncated Fragments of Pasteurella Multocida Toxin and Their Immunogenicity' CANADIAN JOURNAL OF VETERINARY RESEARCH vol. 3, no. 73, 31 July 2009, XP055287040**
- **LIAN, YANXIAN ET AL.: 'Construction of DNA Vaccines Expressing Fusion of C3d-HA Against Swine Influenza Virus' PROGRESS IN VETERINARY MEDICINE vol. 6, no. 32, 31 December 2011, XP008181243**

SEQUENCE LISTING

<110> SBC Virbac Limited

<120> Recombinant Pasteurella multocida Toxin And Application Thereof

<130> P15-0197

<150> CN 201310095061.6
<151> 2013-03-22

<150> PCT/CN2014/073851
<151> 2014-03-21

<160> 42

<170> PatentIn version 3.5

<210> 1
<211> 1281
<212> PRT
<213> Pasteurella multocida

<300>
<308> GenBank: ABR23002
<309> 2007-08-13
<313> (2)..(1282)

<400> 1


Lys Thr Lys His Phe Phe Asn Ser Asp Phe Thr Val Lys Gly Lys Ser
1               5                   10                  15


Ala Asp Glu Ile Phe Arg Arg Leu Cys Thr Asp His Pro Asp Lys Gln
20                  25                  30


Leu Asn Asn Val Lys Trp Lys Glu Val Phe Ile Asn Arg Phe Gly Gln       35
40                  45


Met Met Leu Asp Thr Pro Asn Pro Arg Lys Ile Val Glu Lys Ile Ile   50
55                  60


Asn Glu Gly Leu Glu Lys Gln Gly Leu Lys Asn Ile Asp Pro Glu Thr
65                  70                  75                  80


Thr Tyr Phe Asn Ile Phe Ser Ser Ser Asp Ser Ser Asp Gly Asn Val
85                  90                  95


Phe His Tyr Asn Ser Leu Ser Glu Ser Tyr Arg Val Thr Asp Ala Cys
100                 105                 110


Leu Met Asn Ile Phe Val Glu Arg Tyr Phe Asp Asp Trp Asp Leu Leu       115
120                 125


Asn Ser Leu Ala Ser Asn Gly Ile Tyr Ser Val Gly Lys Glu Gly Ala   130
135                 140

-

```
Tyr Tyr Pro Asp His Asp Tyr Gly Pro Glu Tyr Asn Pro Val Trp Gly
145             150             155                 160


Pro Asn Glu Gln Ile Tyr His Ser Arg Val Ile Ala Asp Ile Leu Tyr
165             170             175


Ala Arg Ser Val Trp Asp Glu Phe Lys Lys Tyr Phe Met Glu Tyr Trp
180             185             190


Gln Lys Tyr Ala Gln Leu Tyr Thr Glu Met Leu Ser Asp Thr Phe Leu             195
200             205


Ala Met Ala Ile Gln Gln Tyr Thr Arg Gln Thr Leu Thr Asp Glu Gly             210
215             220


Phe Leu Met Val Cys Asn Thr Tyr Tyr Gly Asn Lys Glu Glu Val Gln
225             230             235                 240


Ile Thr Leu Leu Asp Ile Tyr Gly Tyr Pro Ser Thr Asp Ile Ile Cys
245             250             255


Ile Glu Gln Lys Gly Leu Pro Thr Pro Lys Val Ile Leu Tyr Ile Pro
260             265             270


Gly Gly Thr Gln Pro Phe Val Glu Phe Leu Asn Thr Asp Asp Leu Lys             275
280             285


Gln Trp Ile Ala Trp His Leu Lys Asp Asn Lys His Met Val Ala Phe             290
295             300


Arg Lys His Phe Ser Leu Lys Gln Arg Gln Glu Gly Glu Thr Phe Thr
305             310             315                 320


Gly Ile Asp Lys Ala Leu Gln Tyr Ile Ala Glu Glu Ser Pro Glu Trp
325             330             335


Pro Ala Asn Lys Tyr Ile Leu Tyr Asn Pro Thr His Leu Glu Thr Glu
340             345             350


Asn Leu Phe Asn Ile Met Met Lys Arg Thr Glu Gln Arg Met Leu Glu             355
360             365


Asp Ser Asp Val Gln Ile Arg Ser Asn Ser Glu Ala Thr Arg Asp Tyr             370
375             380


Ala Leu Ser Leu Leu Glu Thr Phe Ile Ser Gln Leu Ser Ala Ile Asp
```

18

385                    390                    395                    400

Met Leu Val Pro Ala Val Gly Ile Pro Ile Asn Phe Ala Leu Ser Ala
405              410              415

Thr Ala Leu Gly Leu Ser Ser Asp Ile Val Val Asn Gly Asp Ser Tyr
420              425              430

Glu Lys Arg Lys Tyr Gly Ile Gly Ser Leu Val Gln Ser Ala Leu Phe          435
440              445

Thr Gly Ile Asn Leu Ile Pro Val Ile Ser Glu Thr Ala Glu Ile Leu          450
455              460

Ser Ser Phe Ser Arg Thr Glu Glu Asp Ile Pro Ala Phe Phe Thr Glu
465              470              475              480

Glu Gln Ala Leu Ala Gln Arg Phe Glu Ile Val Glu Glu Glu Leu His
485              490              495

Ser Ile Ser Pro Asp Asp Pro Pro Arg Glu Ile Thr Asp Glu Asn Leu
500              505              510

His Lys Ile Arg Leu Val Arg Leu Asn Asn Glu Asn Gln Pro Leu Val          515
520              525

Val Leu Arg Arg Leu Gly Gly Asn Lys Phe Ile Arg Ile Glu Pro Ile          530
535              540

Thr Phe Gln Glu Ile Lys Gly Ser Leu Val Ser Glu Val Ile Asn Pro
545              550              555              560

Val Thr Asn Lys Thr Tyr Tyr Val Ser Asn Ala Lys Leu Leu Gly Gly
565              570              575

Ser Pro Tyr Ser Pro Phe Arg Ile Gly Leu Glu Gly Val Trp Thr Pro
580              585              590

Glu Val Leu Lys Ala Arg Ala Ser Val Ile Gly Lys Pro Ile Gly Glu          595
600              605

Ser Tyr Lys Arg Ile Leu Ala Lys Leu Gln Arg Ile His Asn Ser Asn          610
615              620

Ile Leu Asp Glu Arg Gln Gly Leu Met His Glu Leu Met Glu Leu Ile
625              630              635              640

Asp Leu Tyr Glu Glu Ser Gln Pro Ser Ser Glu Arg Leu Asn Ala Phe
645 650 655

Arg Glu Leu Arg Thr Gln Leu Glu Lys Ala Leu Tyr Leu Pro Glu Met
660 665 670

Glu Ala Leu Lys Lys Gln Ile Leu Gln Ile Pro Asn Lys Gly Ser Gly 675
680 685

Ala Ala Arg Phe Leu Leu Arg Thr Ala Met Asn Glu Met Ala Gly Glu 690
695 700

Thr Ser Glu Ser Thr Ala Asp Leu Ile Arg Phe Ala Leu Gln Asp Thr
705 710 715 720

Val Ile Ser Ala Pro Phe Arg Gly Tyr Ala Gly Ala Ile Pro Glu Ala
725 730 735

Ile Asp Phe Pro Val Lys Tyr Val Ile Glu Asp Ile Ser Val Phe Asp
740 745 750

Lys Ile Gln Thr Asn Tyr Trp Glu Leu Pro Ala Tyr Glu Ser Trp Asn 755
760 765

Glu Gly Ser Asn Ser Ala Leu Leu Pro Gly Leu Leu Arg Glu Ser Gln 770
775 780

Ser Lys Gly Met Leu Ser Lys Cys Arg Ile Ile Glu Asn Ser Leu Tyr
785 790 795 800

Ile Gly His Ser Tyr Glu Glu Met Phe Tyr Ser Ile Ser Pro Tyr Ser
805 810 815

Asn Gln Val Gly Gly Pro Tyr Glu Leu Tyr Pro Phe Thr Phe Phe Ser
820 825 830

Met Leu Gln Glu Val Gln Gly Asp Leu Gly Phe Glu Gln Ala Phe Ala 835
840 845

Thr Arg Asn Phe Phe Asn Thr Leu Val Ser Asp Arg Leu Ser Leu Met 850
855 860

Glu Asn Thr Met Leu Leu Thr Glu Ser Phe Asp Tyr Thr Pro Trp Asp
865 870 875 880

Ala Ile Tyr Gly Asp Ile Asn Tyr Asp Glu Gln Phe Ala Ala Met Ser
885 890 895

-

```
Ile Asn Glu Arg Ile Glu Lys Cys Met Asn Thr Tyr Arg Gly Val Ala
900             905             910

Phe Gln Asn Ser Ser Lys Ser Ile Asp Phe Phe Leu Asn Asn Leu Thr        915
920             925

Thr Phe Ile Asp Asn Gly Leu Thr Glu Ile Ala Ile Ser Asp Leu Pro     930
935             940

Tyr Asp Ile Val Gln Gln Glu Ile Ser Gln Phe Leu Gln Gly Ser Asn
945             950             955             960

Glu Trp Lys Thr Leu Asp Ala Met Leu Phe Asn Leu Asp Lys Gly Asp
965             970             975

Ile Asn Gly Ala Phe Arg Lys Leu Leu Gln Ser Ala Lys Asp Asn Asn
980             985             990

Ile Lys Phe Arg Ala Ile Gly His  Ser Asp Asn Ser Val  Pro Pro Phe
995             1000            1005

Asn Asn  Pro Tyr Lys Ser Leu  Tyr Tyr Lys Gly Asn  Ile Ile Ala      1010
1015            1020

Glu Ala  Ile Glu Lys Leu Asp  Arg Glu Gly Gln Lys  Phe Val Val      1025
1030            1035

Phe Ala  Asp Ser Ser Leu Leu  Asn Ser Thr Pro Gly  Thr Gly Arg      1040
1045            1050

Pro Met  Pro Gly Leu Val Gln  Tyr Leu Lys Ile Pro  Ala Thr Val      1055
1060            1065

Val Asp  Ser Asp Gly Ala Trp  Gln Phe Leu Pro Asp  Val Ala Ser      1070
1075            1080

Ser Arg  Val Pro Ile Glu Val  Thr Glu Leu Glu Asn  Trp Gln Val      1085
1090            1095

Leu Thr  Pro Pro Gln Gly Lys  Ile Leu Gly Leu Lys  Gln Phe Lys      1100
1105            1110

Leu Thr  Ala Gly Phe Pro Thr  Glu Gln Ser Arg Leu  Pro Leu Leu      1115
1120            1125

Glu Asn  Ser Val Ser Glu Asp  Leu Arg Glu Glu Leu  Met Gln Lys      1130
1135            1140
```

21

Ile Asp Ala Ile Lys Asn Asp Val Lys Met Asn Ser Leu Val Cys 1145
1150 1155

Met Glu Ala Gly Ser Cys Asp Ser Val Ser Pro Lys Val Ala Ala 1160
1165 1170

Arg Leu Lys Asp Met Gly Leu Glu Ala Gly Met Gly Ala Ser Ile 1175
1180 1185

Thr Trp Trp Arg Arg Glu Gly Gly Met Glu Phe Ser His Gln Met 1190
1195 1200

His Thr Thr Ala Ser Phe Lys Phe Ala Gly Lys Glu Phe Ala Val 1205
1210 1215

Asp Ala Ser His Leu Gln Phe Val His Asp Gln Leu Asp Thr Thr 1220
1225 1230

Ile Leu Ile Leu Pro Val Asp Asp Trp Ala Leu Glu Ile Ala Gln 1235
1240 1245

Arg Asn Arg Ala Ile Asn Pro Phe Val Glu Tyr Val Ser Lys Thr 1250
1255 1260

Gly Asn Met Leu Ala Leu Phe Met Pro Pro Leu Phe Thr Lys Pro 1265
1270 1275

Arg Leu Thr 1280

<210> 2
<211> 79
<212> PRT
<213> Pasteurella multocida

<300>
<308> GenBank: ABR23002
<309> 2007-08-13
<313> (87)..(165)

<400> 2

```
Phe Ser Ser Ser Asp Ser Ser Asp Gly Asn Val Phe His Tyr Asn Ser
1               5               10              15
```

```
Leu Ser Glu Ser Tyr Arg Val Thr Asp Ala Cys Leu Met Asn Ile Phe
20              25              30
```

```
Val Glu Arg Tyr Phe Asp Asp Trp Asp Leu Leu Asn Ser Leu Ala Ser        35
40              45
```

```
Asn Gly Ile Tyr Ser Val Gly Lys Glu Gly Ala Tyr Tyr Pro Asp His        50
55              60
```

```
Asp Tyr Gly Pro Glu Tyr Asn Pro Val Trp Gly Pro Asn Glu Gln
65              70              75
```

<210> 3
<211> 75
<212> PRT
<213> Pasteurella multocida

<300>
<308> GenBank: ABR23002
<309> 2007-08-13
<313> (1001)..(1075)

<400> 3

```
His Ser Asp Asn Ser Val Pro Pro Phe Asn Asn Pro Tyr Lys Ser Leu
1               5               10              15
```

```
Tyr Tyr Lys Gly Asn Ile Ile Ala Glu Ala Ile Glu Lys Leu Asp Arg
20              25              30
```

```
Glu Gly Gln Lys Phe Val Val Phe Ala Asp Ser Ser Leu Leu Asn Ser        35
40              45
```

```
Thr Pro Gly Thr Gly Arg Pro Met Pro Gly Leu Val Gln Tyr Leu Lys        50
55              60
```

```
Ile Pro Ala Thr Val Val Asp Ser Asp Gly Ala
65              70              75
```

<210> 4
<211> 59
<212> PRT
<213> Pasteurella multocida

<300>
<308> GenBank: ABR23002
<309> 2007-08-13
<313> (1045)..(1103)

<400> 4

Leu Leu Asn Ser Thr Pro Gly Thr Gly Arg Pro Met Pro Gly Leu Val
1               5                   10                  15

Gln Tyr Leu Lys Ile Pro Ala Thr Val Val Asp Ser Asp Gly Ala Trp
20                  25                  30

Gln Phe Leu Pro Asp Val Ala Ser Ser Arg Val Pro Ile Glu Val Thr          35

40                      45

Glu Leu Glu Asn Trp Gln Val Leu Thr Pro Pro          50                      55

<210> 5
<211> 14
<212> PRT
<213> Artificial Sequence

<220>
<223> Amino acid sequence of linker for PMT B-cell epitopes

<400> 5

Gly Ser Gly Gly Gly Gly Ser Gly Gly Gly Gly Ser Gly Ser
1               5                   10

<210> 6
<211> 28
<212> PRT
<213> Mus musculus

<300>
<308> GenBank: ADL70201
<309> 2010-08-18
<313> (211)..(238)

<400> 6

Lys Phe Leu Asn Thr Ala Lys Asp Arg Asn Arg Trp Glu Glu Pro Asp
1               5                   10                  15

Gln Gln Leu Tyr Asn Val Glu Ala Thr Ser Tyr Ala          20
25

<210> 7
<211> 31
<212> PRT
<213> Sus scrofa

<300>
<308> GenBank: ADG26759
<309> 2010-05-08

<313> (201)..(231)

<400> 7

```
Phe Leu Asn Lys Leu Leu Ser Thr Ala Lys Glu Arg Asn Arg Trp Glu
1               5                   10                  15


Glu Pro Gly Gln Lys Leu Tyr Asn Val Glu Ala Thr Ser Tyr Ala          20
    25              30
```

<210> 8
<211> 312
<212> PRT
<213> Mus musculus

<300>
<308> GenBank: ADL70201
<309> 2010-08-18
<313> (1)..(312)

<400> 8

```
Arg Leu Lys His Leu Ile Val Thr Pro Ala Gly Cys Gly Glu Gln Asn
1               5               10              15


Met Ile Gly Met Thr Pro Thr Val Ile Ala Val His Tyr Leu Asp Gln
20              25              30                                  35


Thr Glu Gln Trp Glu Lys Phe Gly Ile Glu Lys Arg Gln Glu Ala Leu   50
40              45


Glu Leu Ile Lys Lys Gly Tyr Thr Gln Gln Leu Ala Phe Lys Gln Pro
55              60


Ser Ser Ala Tyr Ala Ala Phe Asn Asn Arg Pro Pro Ser Thr Trp Leu
65              70              75              80


Thr Ala Tyr Val Val Lys Val Phe Ser Leu Ala Ala Asn Leu Ile Ala
85              90              95


Ile Asp Ser His Val Leu Cys Gly Ala Val Lys Trp Leu Ile Leu Glu
100             105             110


Lys Gln Lys Pro Asp Gly Val Phe Gln Glu Asp Gly Pro Val Ile His   115
120             125


Gln Glu Met Ile Gly Gly Phe Arg Asn Ala Lys Glu Ala Asp Val Ser   130
135             140


Leu Thr Ala Phe Val Leu Ile Ala Leu Gln Glu Ala Arg Asp Ile Cys
145             150             155             160


Glu Gly Gln Val Asn Ser Leu Pro Gly Ser Ile Asn Lys Ala Gly Glu
165             170             175


Tyr Ile Glu Ala Ser Tyr Met Asn Leu Gln Arg Pro Tyr Thr Val Ala
180             185             190


Ile Ala Gly Tyr Ala Leu Ala Leu Met Asn Lys Leu Glu Glu Pro Tyr   195
200             205


Leu Gly Lys Phe Leu Asn Thr Ala Lys Asp Arg Asn Arg Trp Glu Glu   210
```

Pro Asp Gln Gln Leu Tyr Asn Val Glu Ala Thr Ser Tyr Ala Leu Leu

Ala Leu Leu Leu Leu Lys Asp Phe Asp Ser Val Pro Pro Val Val Arg

Trp Leu Asn Glu Gln Arg Tyr Tyr Gly Gly Gly Tyr Gly Ser Thr Gln

Ala Thr Phe Met Val Phe Gln Ala Leu Ala Gln Tyr Gln Thr Asp Val

Pro Asp His Lys Asp Leu Asn Met Asp Val Ser Phe His Leu Pro Ser

Cys Ser Ser Ala Thr Thr Phe Arg

<210> 9
<211> 296
<212> PRT
<213> Sus scrofa

<300>
<308> GenBank: ADG26759
<309> 2010-05-08
<313> (1)..(296)

<400> 9

```
Thr Pro Ser Gly Cys Gly Lys Gln Asn Met Ile Gly Met Thr Pro Thr
1               5                   10                  15


Val Ile Ala Val His Tyr Leu Asp Ser Thr Glu Gln Trp Glu Lys Phe
20              25                  30


Gly Leu Glu Lys Arg Gln Glu Ala Leu Glu Leu Ile Lys Lys Gly Tyr        35
40              45


Thr Gln Gln Leu Ala Phe Arg Gln Lys Asn Ser Ala Phe Ala Ala Phe     50
55              60


Gln Asp Arg Leu Ser Ser Thr Trp Leu Thr Ala Tyr Val Val Lys Val
65              70                  75                  80


Phe Ala Met Ala Ala Asn Leu Ile Ala Ile Asp Ser Gln Val Leu Cys
85              90                  95
```

```
Gly Ala Val Lys Trp Leu Ile Leu Glu Lys Gln Lys Pro Asp Gly Val
100             105             110

Phe Glu Glu Asn Gly Pro Val Ile His Gln Glu Met Ile Gly Gly Phe      115
120             125

Lys Asn Thr Glu Glu Lys Asp Val Ser Leu Thr Ala Phe Val Leu Ile      130
135             140

Ala Leu Gln Glu Ala Lys Asp Ile Cys Glu Pro Gln Val Asn Ser Leu
145             150             155             160

Leu Arg Ser Ile Asn Lys Ala Arg Asp Phe Leu Ala Asp Tyr Tyr Leu
165             170             175

Glu Leu Lys Arg Pro Tyr Thr Val Ala Ile Ala Gly Tyr Ala Leu Ala
180             185             190

Leu Ser Asp Lys Leu Asp Glu Pro Phe Leu Asn Lys Leu Leu Ser Thr    195
200             205

Ala Lys Glu Arg Asn Arg Trp Glu Glu Pro Gly Gln Lys Leu Tyr Asn    210
215             220

Val Glu Ala Thr Ser Tyr Ala Leu Leu Ala Leu Leu Val Val Lys Asp
225             230             235             240

Phe Asp Ser Val Pro Pro Ile Val Arg Trp Leu Asn Glu Gln Arg Tyr
245             250             255

Tyr Gly Gly Gly Tyr Gly Ser Thr Gln Ala Thr Phe Met Val Phe Gln
260             265             270

Ala Leu Ala Gln Tyr Gln Lys Asp Val Pro Asp His Lys Asp Leu Asn    275
280             285

Leu Asp Val Ser Ile His Leu Pro      290                295
```

<210> 10
<211> 441
<212> PRT
<213> Artificial Sequence

<220>
<223> Amino acid sequence of recombinant Pasteurella multocida toxin 1 (rPMT1)

<400> 10

Phe Ser Ser Ser Asp Ser Ser Asp Gly Asn Val Phe His Tyr Asn Ser

EP 2 982 683 B1

|  | 1 | | | 5 | | | 10 | | | 15 | |
|---|---|---|---|---|---|---|---|---|---|---|

Leu Ser Glu Ser Tyr Arg Val Thr Asp Ala Cys Leu Met Asn Ile Phe
20        25        30

Val Glu Arg Tyr Phe Asp Asp Trp Asp Leu Leu Asn Ser Leu Ala Ser        35
40        45

Asn Gly Ile Tyr Ser Val Gly Lys Glu Gly Ala Tyr Tyr Pro Asp His        50
55        60

Asp Tyr Gly Pro Glu Tyr Asn Pro Val Trp Gly Pro Asn Glu Gln Gly
65        70        75        80

Ser Gly Gly Gly Gly Ser Gly Gly Gly Gly Ser Gly Ser His Ser Asp
85        90        95

Asn Ser Val Pro Pro Phe Asn Asn Pro Tyr Lys Ser Leu Tyr Tyr Lys
100        105        110

Gly Asn Ile Ile Ala Glu Ala Ile Glu Lys Leu Asp Arg Glu Gly Gln        115
120        125

Lys Phe Val Val Phe Ala Asp Ser Ser Leu Leu Asn Ser Thr Pro Gly        130
135        140

Thr Gly Arg Pro Met Pro Gly Leu Val Gln Tyr Leu Lys Ile Pro Ala
145        150        155        160

Thr Val Val Asp Ser Asp Gly Ala Gly Ser Gly Gly Gly Gly Ser Gly
165        170        175

Gly Gly Gly Ser Gly Ser Leu Leu Asn Ser Thr Pro Gly Thr Gly Arg
180        185        190

Pro Met Pro Gly Leu Val Gln Tyr Leu Lys Ile Pro Ala Thr Val Val        195
200        205

Asp Ser Asp Gly Ala Trp Gln Phe Leu Pro Asp Val Ala Ser Ser Arg        210
215        220

Val Pro Ile Glu Val Thr Glu Leu Glu Asn Trp Gln Val Leu Thr Pro
225        230        235        240

Pro Gly Ser Gly Gly Gly Gly Ser Gly Gly Gly Gly Ser Gly Ser Arg
245        250        255

31

```
Ser Pro Gly Gly Ser Lys Phe Leu Asn Thr Ala Lys Asp Arg Asn Arg
260             265             270


Trp Glu Glu Pro Asp Gln Gln Leu Tyr Asn Val Glu Ala Thr Ser Tyr       275
280             285


Ala Gly Ser Lys Phe Leu Asn Thr Ala Lys Asp Arg Asn Arg Trp Glu    290
295             300


Glu Pro Asp Gln Gln Leu Tyr Asn Val Glu Ala Thr Ser Tyr Ala Gly
305             310             315             320


Ser Lys Phe Leu Asn Thr Ala Lys Asp Arg Asn Arg Trp Glu Glu Pro
325             330             335


Asp Gln Gln Leu Tyr Asn Val Glu Ala Thr Ser Tyr Ala Gly Ser Lys
340             345             350


Phe Leu Asn Thr Ala Lys Asp Arg Asn Arg Trp Glu Glu Pro Asp Gln       355
360             365


Gln Leu Tyr Asn Val Glu Ala Thr Ser Tyr Ala Gly Ser Lys Phe Leu    370
375             380


Asn Thr Ala Lys Asp Arg Asn Arg Trp Glu Glu Pro Asp Gln Gln Leu
385             390             395             400


Tyr Asn Val Glu Ala Thr Ser Tyr Ala Gly Ser Lys Phe Leu Asn Thr
405             410             415


Ala Lys Asp Arg Asn Arg Trp Glu Glu Pro Asp Gln Gln Leu Tyr Asn
420             425             430


Val Glu Ala Thr Ser Tyr Ala Gly Ser        435                 440
```

<210> 11
<211> 459
<212> PRT
<213> Artificial Sequence

<220>
<223> Amino acid sequence of recombinant Pasteurella multocida toxin 2
(rPMT2)

<400> 11

Phe Ser Ser Ser Asp Ser Ser Asp Gly Asn Val Phe His Tyr Asn Ser
1               5               10                  15

Leu Ser Glu Ser Tyr Arg Val Thr Asp Ala Cys Leu Met Asn Ile Phe

```
                20                          25                          30

        Val Glu Arg Tyr Phe Asp Asp Trp Asp Leu Leu Asn Ser Leu Ala Ser        35
        40                          45

        Asn Gly Ile Tyr Ser Val Gly Lys Glu Gly Ala Tyr Tyr Pro Asp His     50
        55                          60

        Asp Tyr Gly Pro Glu Tyr Asn Pro Val Trp Gly Pro Asn Glu Gln Gly
        65                          70                          75                          80

        Ser Gly Gly Gly Gly Ser Gly Gly Gly Gly Ser Gly Ser His Ser Asp
        85                          90                          95

        Asn Ser Val Pro Pro Phe Asn Asn Pro Tyr Lys Ser Leu Tyr Tyr Lys
        100                         105                         110

        Gly Asn Ile Ile Ala Glu Ala Ile Glu Lys Leu Asp Arg Glu Gly Gln        115
        120                         125

        Lys Phe Val Val Phe Ala Asp Ser Ser Leu Leu Asn Ser Thr Pro Gly    130
        135                         140

        Thr Gly Arg Pro Met Pro Gly Leu Val Gln Tyr Leu Lys Ile Pro Ala
        145                         150                         155                         160

        Thr Val Val Asp Ser Asp Gly Ala Gly Ser Gly Gly Gly Gly Ser Gly
        165                         170                         175

        Gly Gly Gly Ser Gly Ser Leu Leu Asn Ser Thr Pro Gly Thr Gly Arg
        180                         185                         190

        Pro Met Pro Gly Leu Val Gln Tyr Leu Lys Ile Pro Ala Thr Val Val        195
        200                         205

        Asp Ser Asp Gly Ala Trp Gln Phe Leu Pro Asp Val Ala Ser Ser Arg     210
        215                         220

        Val Pro Ile Glu Val Thr Glu Leu Glu Asn Trp Gln Val Leu Thr Pro
        225                         230                         235                         240

        Pro Gly Ser Gly Gly Gly Gly Ser Gly Gly Gly Gly Ser Gly Ser Gly
        245                         250                         255

        Thr Gly Ser Gly Ser Phe Leu Asn Lys Leu Leu Ser Thr Ala Lys Glu
        260                         265                         270
```

34

Arg Asn Arg Trp Glu Glu Pro Gly Gln Lys Leu Tyr Asn Val Glu Ala          275
280                 285

Thr Ser Tyr Ala Gly Ser Phe Leu Asn Lys Leu Leu Ser Thr Ala Lys          290
295                 300

Glu Arg Asn Arg Trp Glu Glu Pro Gly Gln Lys Leu Tyr Asn Val Glu
305                 310                 315                 320

Ala Thr Ser Tyr Ala Gly Ser Phe Leu Asn Lys Leu Leu Ser Thr Ala
325                 330                 335

Lys Glu Arg Asn Arg Trp Glu Glu Pro Gly Gln Lys Leu Tyr Asn Val
340                 345                 350

Glu Ala Thr Ser Tyr Ala Gly Ser Phe Leu Asn Lys Leu Leu Ser Thr          355
360                 365

Ala Lys Glu Arg Asn Arg Trp Glu Glu Pro Gly Gln Lys Leu Tyr Asn          370
375                 380

Val Glu Ala Thr Ser Tyr Ala Gly Ser Phe Leu Asn Lys Leu Leu Ser
385                 390                 395                 400

Thr Ala Lys Glu Arg Asn Arg Trp Glu Glu Pro Gly Gln Lys Leu Tyr
405                 410                 415

Asn Val Glu Ala Thr Ser Tyr Ala Gly Ser Phe Leu Asn Lys Leu Leu
420                 425                 430

Ser Thr Ala Lys Glu Arg Asn Arg Trp Glu Glu Pro Gly Gln Lys Leu          435
440                 445

Tyr Asn Val Glu Ala Thr Ser Tyr Ala Gly Ser          450                 455

<210> 12
<211> 6
<212> PRT
<213> Artificial Sequence

<220>
<223> Amino acid sequence of (GS)3 linker

<400> 12

Gly Ser Gly Ser Gly Ser
1                 5

<210> 13

<211> 15
<212> PRT
<213> Artificial Sequence

<220>
<223> amino acid sequence of (GGGGS)3 linker

<400> 13

```
        Gly Gly Gly Gly Ser Gly Gly Gly Gly Ser Gly Gly Gly Gly Ser
        1               5                   10                  15
```

<210> 14
<211> 4
<212> PRT
<213> Artificial Sequence

<220>
<223> amino acid sequence of GGSG linker

<400> 14

```
                        Gly Gly Ser Gly
                        1
```

<210> 15
<211> 8
<212> PRT
<213> Artificial Sequence

<220>
<223> Amino acid sequence of (GGSG)2 linker

<400> 15

```
                    Gly Gly Ser Gly Gly Gly Ser Gly
                    1               5
```

<210> 16
<211> 12
<212> PRT
<213> Artificial Sequence

<220>
<223> amino acid sequence of (GGSG)3 linker

<400> 16

```
                Gly Gly Ser Gly Gly Gly Ser Gly Gly Gly Ser Gly
                1               5                   10
```

<210> 17
<211> 10
<212> PRT
<213> Artificial Sequence

<220>
<223> amino acid sequence of GENLYFQSGG linker

<400> 17

```
              Gly Glu Asn Leu Tyr Phe Gln Ser Gly Gly
              1               5                   10
```

<210> 18
<211> 36
<212> PRT
<213> Artificial Sequence

<220>
<223> amino acid sequence of GAST linker

<400> 18

```
        Gly Gly Ser Ala Gly Gly Ser Gly Ser Gly Ser Ser Gly Gly Ser Ser
        1               5               10                  15


        Gly Ala Ser Gly Thr Gly Thr Ala Gly Gly Thr Gly Ser Gly Ser Gly
        20              25                  30


        Thr Gly Ser Gly           35
```

<210> 19
<211> 36
<212> PRT
<213> Artificial Sequence

<220>
<223> amino acid sequence of SEG linker

<400> 19

```
        Gly Gly Ser Gly Gly Gly Ser Glu Gly Gly Gly Ser Glu Gly Gly Gly
        1               5               10                  15


        Ser Glu Gly Gly Gly Ser Glu Gly Gly Gly Ser Glu Gly Gly Gly Ser
        20              25                  30


        Gly Gly Gly Ser           35
```

<210> 20
<211> 8
<212> PRT
<213> Pasteurella multocida

<300>
<308> GenBank: ABR23002
<309> 2007-08-13
<313> (89)..(96)

<400> 20

```
                    Ser Ser Asp Ser Ser Asp Gly Asn
                    1                   5
```

<210> 21
<211> 29
<212> PRT
<213> Pasteurella multocida

<300>
<308> GenBank: ABR23002
<309> 2007-08-13
<313> (138)..(166)

<400> 21

```
        Tyr Ser Val Gly Lys Glu Gly Ala Tyr Tyr Pro Asp His Asp Tyr Gly
        1                   5                   10                  15


        Pro Glu Tyr Asn Pro Val Trp Gly Pro Asn Glu Gln Ile           20
        25
```

<210> 22
<211> 20
<212> PRT
<213> Pasteurella multocida

<300>
<308> GenBank: ABR23002
<309> 2007-08-13
<313> (87)..(106)

<400> 22

```
        Phe Ser Ser Ser Asp Ser Ser Asp Gly Asn Val Phe His Tyr Asn Ser
        1                   5                   10                  15


        Leu Ser Glu Ser                 20
```

<210> 23
<211> 20
<212> PRT
<213> Pasteurella multocida

<300>
<308> GenBank: ABR23002
<309> 2007-08-13
<313> (141)..(160)

<400> 23

```
        Gly Lys Glu Gly Ala Tyr Tyr Pro Asp His Asp Tyr Gly Pro Glu Tyr
        1                   5                   10                  15


        Asn Pro Val Trp                 20
```

<210> 24
<211> 20
<212> PRT
<213> Pasteurella multocida

<300>
<308> GenBank: ABR23002
<309> 2007-08-13
<313> (114)..(133)

<400> 24

```
Leu Met Asn Ile Phe Val Glu Arg Tyr Phe Asp Asp Trp Asp Leu Leu
1               5                   10                  15

Asn Ser Leu Ala             20
```

<210> 25
<211> 14
<212> PRT
<213> Pasteurella multocida

<300>
<308> GenBank: ABR23002
<309> 2007-08-13
<313> (88)..(101)

<400> 25

```
Ser Ser Ser Asp Ser Ser Asp Gly Asn Val Phe His Tyr Asn
1               5                   10
```

<210> 26
<211> 14
<212> PRT
<213> Pasteurella multocida

<300>
<308> GenBank: ABR23002
<309> 2007-08-13
<313> (141)..(154)

<400> 26

```
Gly Lys Glu Gly Ala Tyr Tyr Pro Asp His Asp Tyr Gly Pro
1               5                   10
```

<210> 27
<211> 14
<212> PRT
<213> Pasteurella multocida

<300>
<308> GenBank: ABR23002
<309> 2007-08-13
<313> (119)..(132)

<400> 27

```
        Val Glu Arg Tyr Phe Asp Asp Trp Asp Leu Leu Asn Ser Leu
        1               5                   10
```

<210> 28
<211> 14
<212> PRT
<213> Pasteurella multocida

<300>
<308> GenBank: ABR23002
<309> 2007-08-13
<313> (156)..(169)

<400> 28

```
        Tyr Asn Pro Val Trp Gly Pro Asn Glu Gln Ile Tyr His Ser
        1               5                   10
```

<210> 29
<211> 20
<212> PRT
<213> Pasteurella multocida

<300>
<308> GenBank: ABR23002
<309> 2007-08-13
<313> (140)..(159)

<400> 29

```
        Val Gly Lys Glu Gly Ala Tyr Tyr Pro Asp His Asp Tyr Gly Pro Glu
        1               5                   10                  15

        Tyr Asn Pro Val               20
```

<210> 30
<211> 14
<212> PRT
<213> Pasteurella multocida

<300>
<308> GenBank: ABR23002
<309> 2007-08-13
<313> (1001)..(1014)

<400> 30

```
        His Ser Asp Asn Ser Val Pro Pro Phe Asn Asn Pro Tyr Lys
        1               5                   10
```

<210> 31
<211> 13
<212> PRT
<213> Pasteurella multocida

```
<300>
<308> GenBank: ABR23002
<309> 2007-08-13
<313> (1045)..(1057)

<400> 31
```

```
                                        -
              Leu Leu Asn Ser Thr Pro Gly Thr Gly Arg Pro Met Pro
              1               5                   10
```

```
<210> 32
<211> 8
<212> PRT
<213> Pasteurella multocida
```

```
<300>
<308> GenBank: ABR23002
<309> 2007-08-13
<313> (1068)..(1075)

<400> 32
```

```
                        Thr Val Val Asp Ser Asp Gly Ala
                        1               5
```

```
<210> 33
<211> 20
<212> PRT
<213> Pasteurella multocida
```

```
<300>
<308> GenBank: ABR23002
<309> 2007-08-13
<313> (1049)..(1068)

<400> 33
```

```
        Thr Pro Gly Thr Gly Arg Pro Met Pro Gly Leu Val Gln Tyr Leu Lys
        1               5                   10                  15


        Ile Pro Ala Thr                     20
```

```
<210> 34
<211> 20
<212> PRT
<213> Pasteurella multocida
```

```
<300>
<308> GenBank: ABR23002
<309> 2007-08-13
<313> (1001)..(1020)

<400> 34
```

```
His Ser Asp Asn Ser Val Pro Pro Phe Asn Asn Pro Tyr Lys Ser Leu
1               5                   10                  15


Tyr Tyr Lys Gly              20
```

<210> 35
<211> 14
<212> PRT
<213> Pasteurella multocida

<300>
<308> GenBank: ABR23002
<309> 2007-08-13
<313> (1046)..(1059)

<400> 35

```
Leu Asn Ser Thr Pro Gly Thr Gly Arg Pro Met Pro Gly Leu
1               5                   10
```

<210> 36
<211> 20
<212> PRT
<213> Pasteurella multocida

<300>
<308> GenBank: ABR23002
<309> 2007-08-13
<313> (1048)..(1067)

<400> 36

```
Ser Thr Pro Gly Thr Gly Arg Pro Met Pro Gly Leu Val Gln Tyr Leu
1               5                   10                  15


Lys Ile Pro Ala              20
```

<210> 37
<211> 5
<212> PRT
<213> Pasteurella multocida

<300>
<308> GenBank: ABR23002
<309> 2007-08-13
<313> (1082)..(1086)

<400> 37

```
Val Ala Ser Ser Arg
1               5
```

<210> 38
<211> 7
<212> PRT
<213> Pasteurella multocida

<300>
<308> GenBank: ABR23002
<309> 2007-08-13
<313> (1099)..(1105)

<400> 38

```
                              Val Leu Thr Pro Pro Gln Gly
                              1               5
```

<210> 39
<211> 20
<212> PRT
<213> Pasteurella multocida

<300>
<308> GenBank: ABR23002
<309> 2007-08-13
<313> (1092)..(1111)

<400> 39

```
        Thr Glu Leu Glu Asn Trp Gln Val Leu Thr Pro Pro Gln Gly Lys Ile
        1               5                   10                  15

        Leu Gly Leu Lys                 20
```

<210> 40
<211> 14
<212> PRT
<213> Pasteurella multocida

<300>
<308> GenBank: ABR23002
<309> 2007-08-13
<313> (1080)..(1093)

<400> 40

```
            Pro Asp Val Ala Ser Ser Arg Val Pro Ile Glu Val Thr Glu
            1               5                   10
```

<210> 41
<211> 14
<212> PRT
<213> Pasteurella multocida

<300>
<308> GenBank: ABR23002
<309> 2007-08-13
<313> (1096)..(1109)

<400> 41

```
            Asn Trp Gln Val Leu Thr Pro Pro Gln Gly Lys Ile Leu Gly
            1               5                   10
```

```
<210> 42
<211> 20
<212> PRT
<213> Pasteurella multocida

<300>
<308> GenBank: ABR23002
<309> 2007-08-13
<313> (1086)..(1105)

<400> 42


Arg Val Pro Ile Glu Val Thr Glu Leu Glu Asn Trp Gln Val Leu Thr
1               5                   10                  15

Pro Pro Gln Gly            20
```

**Description**

**BACKGROUND OF THE INVENTION**

**1. FIELD OF THE INVENTION**

The present invention relates to a novel recombinant *Pasteurella multocida* toxin and its application in the field of animal healthcare, particularly to an immunogenic composition against atrophic rhinitis having the recombinant *Pasteurella multocida* toxin and its application in the field of animal healthcare.

**2. DESCRIPTION OF THE PRIOR ART**

Atrophic Rhinitis (AR) is one major respiratory disease in swine. The characteristic signs of this disease include chronic purulent rhinitis, distortion of facial bones and atrophy of the turbinates of affected pigs. In severe cases, lesions can be observed in nasal cavity, chine bone, and maxilla bone. Although the ventral scroll of the ventral turbinates is the most common infection site, dorsal turbinate, ventral turbinate, nasal septum and ethmoid can also be infected.

Atrophic rhinitis is caused by *Bordetella bronchiseptica, Pasteurella multocida* type A (PmA) and type D (PmD), especially *Pasteurella multocida* toxin (PMT) produced by PmD. Intramuscular, intraperitoneal, or intranasal inoculation of PMT in four-week-old piglets all leads to atrophy of turbinates, as well as affecting systemic skeletal development and growth retardation. Inoculation of a high dose of PMT in piglets can even lead to liver damage, jaundice, and death.

Atrophic rhinitis is widespread on most of the pig farms in the world. The disease causes significant global economic loss in swine production due to growth retardation and low feed conversion rates (FCRs) of infected pigs. Although the mortality rate of pigs affected by atrophic rhinitis alone is not high, the disease is highly infectious and is more likely to cause other complications and infection of other pathogen, which leads to high mortality rates and high production cost. The economic loss in pig farms severely affected by atrophic rhinitis is about 15-38%, and growth retardation is significant in those farms, where average daily weigh

The object of the present invention is achieved by a recombinant Pasteurella multocida toxin according to claim 1, a polynucleotide according to claim 3, an immunogenic composition according to claim 4 and a test kit according to claim 9.

The first aspect of the present invention relates to a recombinant *P. multocida* toxin (recombinant PMT, rPMT). The recombinant toxin has at least one epitope of *P. multocida* toxin (PMT) to induce animals to produce anti-PMT antibody. The rPMT may further have at least one unit of partial- or full-length of the amino acid sequence of complement component 3d (C3d) to increase specific immune response. In addition, when the at least one epitope of PMT comprises two or more epitopes, each of the epitopes is linked to another epitope with at least one linker; when the at least one unit of partial- or full-length of the amino acid sequence of complement C3d comprises two or more units, each of the units is linked to another unit with at least one linker; the at least one epitope of PMT is linked to the at least one unit of partial- or full-length of the amino acid sequence of complement C3d with at least one linker.

The rPMT of the present invention may be represented by the formula below:

$$(A)_m\text{-}(C3d\ fragment)_n$$

wherein

each A is an individual epitope of PMT and is independently selected from the group consisting of 2, 3, 4, 20, 21, 22, 23, 24, 25, 26, 27, 28, 29, 30, 31, 32, 33, 34, 35, 36, 37, 38, 39, 40, 41, and 42;
each C3d fragment is an individual unit of the amino acid sequence of complement C3d and is independently selected from the group consisting of SEQ ID NOs: 6, 7, 8, and 9;
m is an integer from 1 to about 30; and
n is an integer from 0 to about 10.

The second aspect of the present invention relates to a nucleotide sequence encoding the rPMT. The rPMT comprises at least one epitope of PMT and 0 to 10 units of the amino acid sequence of complement component 3d (C3d).

The third aspect of the present invention relates to an immunogenic composition against atrophic rhinitis. The immunogenic composition against atrophic rhinitis comprises an rPMT

The object of the present invention is achieved by a recombinant *Pasteurella multocida* toxin according to claim 1, a polynucleotide according to claim 2, an immunogenic composition according to claim 3, an anti-*Pasteurella multocida* type D toxin polyclonal antibody according to claim 7, and a test kit according to claim 8.

The first aspect of the present invention relates to a recombinant *P. multocida* toxin (recombinant PMT, rPMT). The recombinant toxin has three epitopes of *P. multocida* toxin (PMT) to induce animals to produce anti-PMT antibody. The rPMT has 6 units of partial--length of the amino acid sequence of complement component 3d (C3d) to increase specific immune response. In addition, each of the epitopes is linked to another epitope with one linker; each unit of complement C3d is linked to another unit with one linker; the epitopes of PMT is linked to the 6 units of partial-length of the amino acid sequence of complement C3d with one linker.

The rPMT of the present invention consists of an amino acid sequence of SEQ ID NO: 10 or 11.

The second aspect of the present invention relates to a nucleotide sequence encoding the rPMT.

The third aspect of the present invention relates to an immunogenic composition against atrophic rhinitis. The immunogenic composition against atrophic rhinitis comprises an rPMT and a pharmaceutically acceptable vehicle. The rPMT consists of an amino acid sequence of SEQ ID NO: 10 or 11. The immunogenic composition against atrophic rhinitis may further comprise *B. bronchiseptica, P. multocida* Type A (PmA), and *P. multocida* Type D (PmD).

The fourth aspect of the present invention relates to an anti-PmD toxin polyclonal antibody. The antibody is derived from the rPMT of the present invention.

The fifth aspect of the present invention relates to a test kit for atrophic rhinitis. The test kit is used to detect PmD toxin or anti-PmD toxin antibodies in a sample.

The immunogenic composition against atrophic rhinitis of the present invention has the following advantages.

The present invention provides an immunogenic composition against atrophic rhinitis comprising an rPMT. The rPMT comprises three epitopes of PMT and partial-length of the amino acid sequence of complement component 3d (C3d). The rPMT is much shorter than the full-length of PMT, so the recombinant toxin can be relatively easily expressed in a biological expression system with higher yields and lower costs.

Furthermore, because the rPMT of the present invention comprises partial-length of the amino acid sequence of C3d, the recombinant protein can increase specific immune response. Experimental data shows that the immunogenic composition against atrophic rhinitis comprising the rPMT of the present invention improves immunity and efficacy against atrophic rhinitis in animals.

The present invention is described in more detail in the following illustrative examples. Although the examples may represent only selected embodiments of the invention, it should be understood that the following examples are illustrative and not limiting.

## BRIEF DESCRIPTION OF THE DRAWINGS

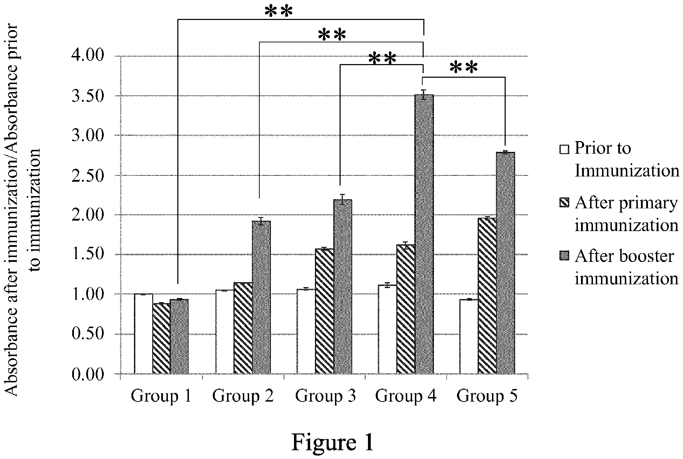

Figure 1 shows ELISA results of antibodies against PMT; Group 1 is the negative control group; Group 2 is immunogenic composition containing *B. bronchiseptica* (B.b), *P. multocida* type A (PmA), and *P. multocida* type D (PmD) obtained in Example 2 (B.b + PmA + PmD group); Group 3 is the rPMT obtained in Example 1 (rPMT group); Group 4 is the immunogenic composition against atrophic rhinitis comprising the rPMT obtained in Example 3 (B.b + PmA + PmD + rPMT group); Group 5 is commercial atrophic rhinitis vaccine (Bayovac® group). ** indicates there is a significant difference between two groups ($p<0.01$).

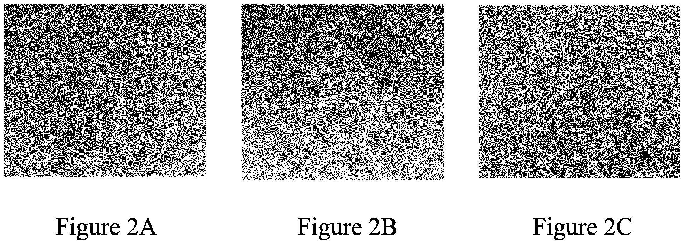

Figure 2 shows the results of neutralization test. Figure 2A shows cell morphology of Vero cells treated with DMEM medium containing FBS (negative control); Figure 2B shows typical nodule-like cytophathic effects (CPE) on Vero cells treated with a four-fold MTD of PMT (positive control); Figure 2C shows cell morphology of Vero cells treated with a neutralized mixture of forty-fold diluted serum from mice inoculated with the immunogenic composition against atrophic rhinitis comprising the rPMT (B.b + PmA + PmD + rPMT group) and a four-fold MTD of PMT.

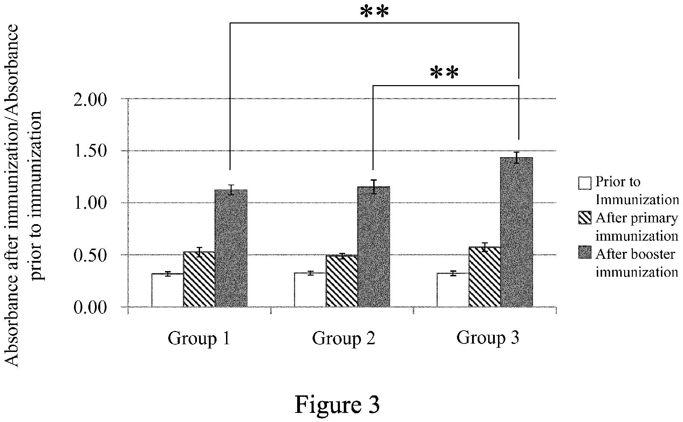

Figure 3 shows ELISA results of antibodies against PMT; Group 1 is the negative control group; Group 2 is immunogenic composition containing *B. bronchiseptica* (B.b), *P. multocida* type A (PmA), and *P. multocida* type D (PmD) obtained in Example 2 (B.b + PmA + PmD group); Group 3 is the immunogenic composition against atrophic rhinitis comprising the rPMT (B.b + PmA+ PmD + rPMT group). ** indicates there is a significant difference between two groups ($p<0.01$).

## DETAILED DESCRIPTION OF THE PREFERRED EMBODIMENT

The present invention relates to an rPMT, which comprises three epitopes of PMT to induce aminals to produce anti-PMT antibody. The three epitopes of PMT are SEQ ID NOs: 2, 3, and 4, respectively.

In a preferred embodiment, each of the epitopes of PMT is linked to another epitope with one linker. The linker has the amino acid sequence of SEQ ID NO: 5.

The rPMT of the present invention further comprises partial-length of the amino acid sequence of C3d to enhance specific immunoresponse. In an embodiment, the full-length of the amino acid sequence of the C3d is the full-length of the amino acid sequence of a mouse complement component 3d (mC3d), as shown in SEQ ID NO: 8. The partial-length of the amino acid sequence of the C3d is the 211th to the 238th amino acids of the mouse C3d (mC3d-p28), which has the sequence of SEQ ID NO: 6. In another embodiment, the full-length of the amino acid sequence of the C3d is the full-length of the amino acid sequence of a pig C3d (pC3d), as shown in SEQ ID NO: 9.

The partial-length of the amino acid sequence of the C3d is the 201st to the 231st amino acids of the pig C3d (pC3d-p31), which has the sequence of SEQ ID NO: 7. The rPMT of the present invention has 6 repeated units of the partial-length of the amino acid sequence of C3d.

Each of the units of the partial-length of the amino acid sequence of C3d is linked to another unit with one linker. The linker is Gly-Ser.

In a preferred embodiment, the rPMT of the present invention has the epitopes covered by SEQ ID NOs: 2, 3, and 4, as well as 6 repeated units of the 211th to the 238th amino acids of the mouse C3d (mC3d-p28). Each of the epitopes is linked to another epitope with a linker (SEQ ID NO: 5), and the rPMT has the amino acid sequence of SEQ ID NO: 10.

In another preferred embodiment, the rPMT of the present invention has the epitopes covered by SEQ ID NOs: 2, 3, and 4, as well as 6 repeated units of the 201st to the 231st amino acids of the pig C3d (pC3d-p31). Each of the epitopes is linked to another epitope with a linker (SEQ ID NO: 5), and the rPMT has the amino acid sequence of SEQ ID NO: 11.

The invention also provides a polynucleotide sequence encoding the rPMT of the present invention. The rPMT comprises three epitopes of PMT and 6 repeated units of the partial-length of the amino acid sequence of C3d. The polynucleotide sequence encoding the rPMT of the present invention is derived from the amino acid sequence of the rPMT by replacing each amino acid with a three-nucleotide codon, including every degenerate codon, listed in the genetic code table below. For example, each serine of the amino acid sequence of the rPMT can be independently encoded by the codons TCT, TCC, TCA, TCG, AGT, or AGC. Each amino acid of the amino acid sequence of the rPMT can be independently encoded by the nucleotide codons below.

| An amino acid of the rPMT | The corresponding nucleotide codons |
|---|---|
| Alanine | GCA, GCC, GCG, GCT |
| Cysteine | TGC, TGT |
| Aspartic acid | GAC, GAT |
| Glutamic acid | GAA, GAG |
| Phenylalanine | TTC, TTT |
| Glycine | GGA, GGC, GGG, GGT |
| Histidine | CAC, CAT |
| Isoleucine | ATA, ATC, ATT |
| Lysine | AAA, AAG |
| Leucine | CTA, CTC, CTG, CTT, TTA, TTG |
| Methionine | ATG |
| Asparagine | AAC, AAT |
| Proline | CCA, CCC, CCG, CCT |
| Glutamine | CAA, CAG |
| Arginine | AGA, AGG, CGA, CGC, CGG, CGT |
| Serine | AGC, AGT, TCA, TCC, TCG, TCT |
| Threonine | ACA, ACC, ACG, ACT |
| Valine | GTA, GTC, GTG, GTT |
| Tryptophan | TGG |
| Tyrosine | TAC, TAT |

In addition, the present invention provides an immunogenic composition against atrophic rhinitis. The immunogenic composition comprises the rPMT of the present invention, which comprises three epitopes of PMT and 6 repeated units of the partial-length of the amino acid sequence of complement component 3d (C3d). In an embodiment, the rPMT has the amino acid sequence of SEQ ID NO: 10. In another embodiment, the rPMT has the amino acid sequence of SEQ ID NO: 11.

The rPMT of the present invention can be synthesized by, but is not limited to, molecular cloning or a peptide synthesizer. The recombinant protein obtained by molecular cloning can be produced by, but is not limited to, inserting a polynucleotide sequence encoding the rPMT into an expression vector to form a plasmid comprising the polynucleotide sequence encoding the rPMT, transforming the plasmid into host cells, and inducing protein expression in the host cells to obtain the recombinant protein.

The expression vector includes, but is not limited to, pET and pGEX expression vectors. The host cells can be, but are not limited to, a prokaryotic cell, such as *Escherichia coli*, or a eukaryotic cell, such as animal cells (insect cells and mammalian cells) and plant cells.

In an embodiment, the immunogenic composition against atrophic rhinitis of the present invention further comprises *B. bronchiseptica, P. multocida* Type A (PmA), and *P. multocida* Type D (PmD). In a preferred embodiment, *B. bronchiseptica, P. multocida* Type A (PmA), and *P. multocida* Type D (PmD) are all obtained from the Animal Health Research Institute, Council of Agriculture, Executive Yuan (Taiwan). However, the sources of *B. bronchiseptica, P. multocida* Type A (PmA), and *P. multocida* Type D (PmD) are not limited to the one mentioned above. *B. bronchiseptica, P. multocida* Type A (PmA), and *P. multocida* Type D (PmD) from other sources, for example, *B. bronchiseptica* ATCC31437 from American Type Culture Collection (ATCC, US) and *P. multocida* Type A (PmA) NCTC 12177 and *P. multocida* Type D (PmD) NCTC 12178 both from National Collection of Type Cultures, (NCTC, UK) or isolation from wild type bacteria, can also be used with the rPMT of the present invention.

The immunogenic composition against atrophic rhinitis of the present invention further comprises at least one pathogen antigen. The pathogen antigens include, but are not limited to, antigen of porcine circovirus type 2 (PCV2), antigen of Swine influenza virus (SIV), antigen of porcine reproductive and respiratory syndrome virus (PRRSV), antigen of mycoplasma, antigen of porcine parvovirus (PPV), antigen of erysipelas, antigen of pseudorabies (Aujeszky's disease) virus, and antigen of *Actinobacillus pleuropneumonia* (App).

The immunogenic composition against atrophic rhinitis of the present invention may further comprise at least one pharmaceutically acceptable vehicles including, but not limited to, solvent, emulsifier, suspending agent, decomposer, binding agent, excipient, stabilizing agent, chelating agent, diluent, gelling agent, preservative, lubricant, surfactant, adjuvant, biological carriers, or other suitable vehicles.

The excipient may be pharmaceutically acceptable organic or inorganic carrier substances suitable for parenteral, enteral or intranasal application which do not deleteriously react with the active compounds and are not deleterious to the recipient thereof. Suitable excipients include, but are not limited to, water, salt solutions, vegetable oils, polyethylene glycols, gelatin, lactose, amylose, magnesium stearate, talc, silicic acid, viscous paraffin, fatty acid monoglycerides and diglycerides, petroethral fatty acid esters, hydroxymethyl-cellulose, polyvinylpyrrolidone, etc.

The pharmaceutically acceptable adjuvant includes, but not limited to, aluminum hydroxide, potassium alum, Freund's Complete Adjuvant, Freund's Incomplete Adjuvant, oil adjuvant (such as mineral oil, plant oil, animal oil etc.), aqueous adjuvant, biphasic emulsification adjuvant (such as water-in-oil-in-water emulsion adjuvant), biological adjuvant (such as CpG oligodeoxynucleotide and toxoid), etc. In an embodiment, the adjuvant is aluminum hydroxide.

The present invention also provides an anti-PmD toxin (PMT) antibody derived from the rPMT of the present invention. In an embodiment, the antibody is a polyclonal antibody obtained via injecting an animal with the rPMT.

The present invention further provides a test kit for atrophic rhinitis. The test kit is used to detect PmD toxin (PMT) or anti-PmD toxin (PMT) antibodies in a test sample. The test kit includes (1) an antigen of the rPMT, in one preferred embodiment, the antigen is deposited on an antigen plate, and/or (2) a polyclonal antibody derived from the rPMT.

The types of the test kit include, but are not limited to, enzyme-linked immunosorbent assay kit (ELISA), microchip kit, immunofluorescent assay (IFA) kit, and other test kits derived from the rPMT. In an embodiment, the test kit comprises at least an antigen plate comprising the rPMT, and the test kit can be used to detect anti-PMT antibodies in a test sample.

As used herein, the singular forms "a", "an", and "the" include plural referents unless the context clearly dictates otherwise. Thus, for example, reference to "a component" includes a plurality of such components and equivalents thereof known to those skilled in the art.

As used herein, "around", "about" or "approximately" shall generally mean within 20 percent, preferably within 10 percent, and more preferably within 5 percent of a given value or range. Numerical quantities given herein are approximate, meaning that the term "around", "about" or "approximately" can be inferred if not expressly stated.

The meaning of the technical and scientific terms as described herein can be clearly understood by a person of ordinary skill in the art.

The present invention is described in more detail in the following illustrative examples.

**EXAMPLES**

**Example 1 Construction** of the **rPMT**

1. Design of the Amino Acid Sequence of the Recombinant *Pasteurella multocida* Toxin 1 (rPMT1)

Epitopes of PMT were predicted on servers for epitope prediction (such as, but not limited to BCPred http://ailab.cs.iastate.edu/bcpreds/) by using the full-length of amino acid sequence of PMT (SEQ ID NO: 1) as the template.
Sequences (SEQ ID NOs: 20 - 42) of the predicted epitopes are listed in Table 1. These epitopes were classified into three fragments, which were Epi 1 (SEQ ID NO: 2), Epi 2 (SEQ ID NO: 3), and Epi 3 (SEQ ID NO: 4), respectively. Table 1 shows the epitopes included in Epi 1, Epi 2, and Epi 3, respectively.

Table 1 Predicting Outcomes of epitopes of *Pasteurella multocida* toxin (PMT)

| Fragments | Sequence | SEQ ID NO. |
|---|---|---|
| Epi1 | SSDSSDGN | 20 |
| Epi 1 | YSVGKEGAYYPDHDYGPEYNPVWGPNEQI | 21 |
| Epi 1 | FSSSDSSDGNVFHYNSLSES | 22 |
| Epi 1 | GKEGAYYPDHDYGPEYNPVW | 23 |
| Epi 1 | LMNIFVERYFDDWDLLNSLA | 24 |
| Epi 1 | SSSDSSDGNVFHYN | 25 |
| Epi 1 | GKEGAYYPDHDYGP | 26 |
| Epi 1 | VERYFDDWDLLNSL | 27 |
| Epi 1 | YNPVWGPNEQIYHS | 28 |
| Epi 1 | VGKEGAYYPDHDYGPEYNPV | 29 |
| Epi 2 | HSDNSVPPFNNPYK | 30 |
| Epi 2 | LLNSTPGTGRPMP | 31 |
| Epi 2 | TVVDSDGA | 32 |
| Epi 2 | TPGTGRPMPGLUQYLKIPAT | 33 |
| Epi 2 | HSDNSVPPFNNPYKSLYYKG | 34 |
| Epi 2 | LNSTPGTGRPMPGL | 35 |
| Epi 2 | STPGTGRPMPGLVQYLKIPA | 36 |
| Epi 3 | LLNSTPGTGRPMP | 31 |
| Epi 3 | TVVDSDGA | 32 |
| Epi 3 | VASSR | 37 |
| Epi 3 | VLTPPQG | 38 |
| Epi 3 | TPGTGRPMPGLUQYLKIPAT | 33 |
| Epi 3 | TELENWQVLTPPQGKILGLK | 39 |
| Epi 3 | PDVASSRVPIEVTE | 40 |
| Epi 3 | LNSTPGTGRPMPGL | 35 |
| Epi 3 | NWQVLTPPQGKILG | 41 |
| Epi 3 | STPGTGRPMPGLVQYLKIPA | 36 |
| Epi 3 | RVPIEVTELENWQVLTPPQG | 42 |

RPMT was designed with different combinations of Epi 1, Epi 2, and Epi 3. A linker sequence (SEQ ID NO: 5)

7

was added to each of the C-termini of Epi 1 (SEQ ID NO: 2), Epi 2 (SEQ ID NO: 3), and Epi 3 (SEQ ID NO: 4). Epi 1, Epi 2, and Epi 3 were linked from N- to C- terminus. Six (6) repeats of mC3d-p28 bioadjuvant (SEQ ID NO: 6) were added to the C-terminus of the linker of Epi 3, in which each mC3d-p28 bioadjuvant was linked to another mC3d-p28 bioadjuvant with a GS linker. The designed recombinant PMT1 (rPMT1) has the amino acid sequence of SEQ ID NO: 10, which was synthesized either by a peptide synthesizer or by molecular cloning.

2. Design of the Amino Acid Sequence of the Recombinant *Pasteurella multocida* Toxin 2 (rPMT2)

Prediction of epitopes of PMT is described above. Epi 1, Epi 2, and Epi 3 were used to design recombinant PMT 2 (rPMT2). A linker sequence (SEQ ID NO: 5) was added to each of the C-termini of Epi 1 (SEQ ID NO: 2), Epi 2 (SEQ ID NO: 3), and Epi 3 (SEQ ID NO: 4). Epi 1, Epi 2, and Epi 3 were linked from N- to C- terminus. Six (6) repeats of pC3d-p31 bioadjuvant (SEQ ID NO: 7) were added to the C-terminus of the linker of Epi 3, in which each pC3d-p31 bioadjuvant was linked to another pC3d-p31 bioadjuvant with a GS linker. The designed rPMT2 has the amino acid sequence of SEQ ID NO: 11, which was synthesized either by a peptide synthesizer or by molecular cloning.

## Example 2 Cultivation of *B. bronchiseptica* and *P. multocida*

### 1. Cultivation of *B. bronchiseptica*

In this example, *B. bronchiseptica* was obtained from the Animal Health Research Institute, Council of Agriculture, Executive Yuan (Taiwan).

*B. bronchiseptica* was cultivated on TSB plates (containing 5% (v/v) yeast extract, 10% (v/v) serum, and tryptic soy broth, TSB, BD Biosciences, USA) overnight at 37°C. After that, a single colony was inoculated in brain heart infusion (BHI) broth medium (BD Biosciences, USA) and incubated overnight at 37°C with shaking. After colony forming unit (CFU) was calculated, bacteria were inactivated with formaldehyde for 24 to 36 hours at room temperature.

### 2. Cultivation of *P. multocida*

In this example, both *P. multocida* type A (PmA) and *P. multocida* type D (PmD) were obtained from the Animal Health Research Institute, Council of Agriculture, Executive Yuan (Taiwan), and the PmD is toxigenic.

PmA and PmD were cultivated on TSB plates (containing 5% (v/v) yeast extract, 10% (v/v) serum, and tryptic soy broth, TSB, BD Biosciences, USA) overnight at 37°C, respectively. After that, a single colony was inoculated in brain heart infusion (BHI) broth medium (BD Biosciences, USA) and incubated overnight at 37°C with shaking. Then, 0.1% (v/v) of the incubated medium was inoculated in BHI broth medium again and incubated overnight at 37°C with shaking. After colony forming unit (CFU) was calculated, bacteria were inactivated with formaldehyde.

## Example 3 Preparation of an Immunogenic Composition Against Atrophic Rhinitis Comprising the rPMT

The rPMT obtained in Example 1 (SEQ ID NO: 10 or 11)(with a final concentration of $65\mu g/ml$) and the inactivated *B. bronchiseptica* (with a final concentration of $1 \times 10^9$ CFU/ml), the inactivated PmA (with a final concentration of $1 \times 10^9$ CFU/ml), and the inactivated PmD (with a final concentration of $1 \times 10^9$ CFU/ml) obtained in Example 2 were mixed in phosphate buffered solution (PBS). Aluminum hydroxide gel was added to final concentration of 30% (v/v) in the mixture as an adjuvant of the immunogenic composition against atrophic rhinitis.

## Example 4 Immunogenicity Assay of the Immunogenic Composition Against Atrophic Rhinitis Comprising the rPMT - Mouse Immunogenicity Test

### 1. Mouse Model

Twenty-six (26) four-week-old Balb/c mice (The National Laboratory Animal Center, Taiwan) were used to conduct the immunogenicity assay. Enzyme-linked immunosorbent assay (ELISA) test showed that all the 26 mice were negative for anti-PMT antibodies, anti-*B. bronchiseptica* antibodies, anti-PmA antibodies, and anti-PmD antibodies.

### 2. Vaccination Program

Those mice were randomly divided into 5 groups, in which 5 mice for each vaccine group and 6 mice for the negative control group. Each mouse was injected intraperitoneally with 0.2 ml of the following substance, respectively:

Group 1: PBS solution with 30% (v/v) of aluminum hydroxide gel (negative control);
Group 2: immunogenic composition containing *B. bronchiseptica* (1x10$^9$ CFU/ml), PmA (1x10$^9$ CFU/ml), and PmD (1x10$^9$ CFU/ml) obtained in Example 2, and 30% (v/v) of aluminum hydroxide gel as adjuvant (B.b + PmA + PmD group);
Group 3: the rPMT obtained in Example 1 (SEQ ID NO: 10, 65μg/ml)(rPMT group);
Group 4: the immunogenic composition against atrophic rhinitis comprising the rPMT (SEQ ID NO: 10) obtained in Example 3 (B.b + PmA + PmD + rPMT group); and
Group 5: commercial atrophic rhinitis vaccine (Bayovac®, Bayer Taiwan Company Ltd., Taiwan)(Bayovac® group).

Fourteen (14) days after primary immunization, each mouse was boosted with the same dose of the substance, respectively. Serum samples were collected 24 hours prior to primary immunization, 13 days after primary immunization, and 10 days after booster immunization. All the serum samples were tested by ELISA.

3. Enzyme-linked immunosorbent assay (ELISA) of toxin antibodies

Commerical PMT (Merck, USA) was coated on 96-well ELISA plates as antigen for 16 hours at 4°C. Uncoated antigen was removed, and the ELISA plates were washed 3 times with wash buffer (0.9% NaCl and 0.1% Tween 20) and air-dried. To block the ELISA plates, blocking solution (wash buffer containing 1% BSA) was added to the ELISA plates, and then the ELISA plates were incubated for an hour at room temperature. After that, the ELISA plates were washed with wash buffer. Mouse serum samples were diluted with PBS and added to the wells of the ELISA plates, and the plates were incubated for an hour at room temperature. After incubation, the serum samples were removed, and the plates were washed with PBS. Secondary antibody conjugated to horseradish peroxidase (HRP)(goat anti-mouse antibody conjugated HRP, Gene Tex Inc., USA) was diluted 5000-fold with blocking solution and then added to the wells (100 μl/well). After incubating for an hour at room temperature, the secondary antibody was removed, and the plates were washed with wash buffer. For visualization of results, 100 μl of 3, 3', 5, 5'-tetramethylbenzidine (TMB, KPL Inc., USA) was added to each well, and the plates were incubated for 10 minutes in dark. Optical density at 650nm (OD650) was read with an ELISA plate reader (SpectraMax® M2/M2 ELISA Reader, Molecular Devices, LLC., USA).
ELISA results are shown in Figure 1. The immunogenic composition against atrophic rhinitis comprising the rPMT (Group 4, B.b + PmA + PmD + rPMT group) induced significantly higher level of serum antibodies against PMT in tested animals than other groups did (*p*<0.01). In addition, commercial atrophic rhinitis vaccine (Group 5, Bayovac group) and the rPMT obtained in Example 1 (Group 3, rPMT group) induced higher level of serum antibodies against PMT in tested animals than Group 1 (control) and Group 2 (B.b + PmA + PmD group). Therefore, the results indicated that the rPMT of the present invention is able to effectively induce immune responses against PMT in mice.

4. Neutralizing Antibody Assay

Vero cells were used to test whether the antibodies against PMT induced by the immunogenic composition against atrophic rhinitis comprising the rPMT (Group 4, B.b + PmA + PmD + rPMT group) can neutralize toxicity of PMT in the cells. Minimum toxin dose (MTD) of Vero cells was measured before neutralizing antibody assay.

(a) Measurement of Minimum Toxin Dose (MTD) of Vero Cells

Vero cells were grown on a 96-well culture plate to reach a confluent monolayer. After that, cell culture medium was discarded, and Vero cells cultured in serum-free DMEM medium (GIBCO, USA) and treated with 2-fold serial dilutions of PMT were observed for cytopathic effect (CPE), whereas Vero cells cultured in DMEM medium containing fetal bovine serum (FBS) were used as negative control. The minimum concentration of PMT that can cause CPE was defined as the minimum toxin dose (MTD). The results indicate that the MTD of PMT in Vero cells is 60ng.

(b) Titration of Neutralizing Antibody

Methods for titration of neutralizing antibodies described by Liao et al. (2006) and Lee et al. (2012) were modified in this Example. (Liao, C. M., Huang, C., Hsuan, S. L., Chen, Z. W., Lee, W. C., Liu, C. I., Winton, J. R., and Chien, M. S. (2006). Immunogenicity and efficacy of three recombinant subunit Pasteurella multocida toxin vaccines against progressive atrophic rhinitis in pigs. Vaccine. 24, 27-35 ; Lee, J., Kang, H. E., and Woo, H. J. (2012). Protective immunity conferred by the C-terminal fragment of recombinant Pasteurella multocida toxin. Clin Vaccine Immunol. 19, 1526-1531.) Serum from mice inoculated with the immunogenic composition against atrophic rhinitis comprising the rPMT (Group 4, B.b + PmA+ PmD + rPMT group) was firstly diluted ten-fold and then two-fold serially (2$^{-1}$~2$^{-6}$) . The diluted mouse serum was added in a 96-well culture plate and incubated with a four-fold MTD of PMT for 1 hour at 37°C. Then, the mixture of the diluted serum and PMT was added to Vero cells in a 96-well culture plate. The plate was incubated at 37°C, 5% $CO_2$ for observation of the ability of the serum to inhibit PMT induced CPE.

The results of neutralization test are shown in Figure 2. Figure 2A shows normal cell morphology of Vero cells treated with DMEM medium containing FBS (negative control), whereas Figure 2B shows typical nodule-like cytophathic effects (CPE) on Vero cells treated with a four-fold MTD of PMT (positive control). Figure 2C shows the cell morphology of Vero cells treated with a neutralized mixture of forty-fold diluted serum from mice inoculated with the immunogenic composition against atrophic rhinitis comprising the rPMT (Group 4, B.b + PmA+ PmD + rPMT group) and a four-fold MTD of PMT. No classic nodular cell surfaces of Vero cells is shown in Figure 2A or Figure 2C. The results indicate that serum from mice inoculated with the immunogenic composition against atrophic rhinitis comprising the rPMT (Group 4, B.b + PmA + PmD + rPMT group) contains neutralizing antibodies against PMT.

**Example 5 Immunogenicity and Protection Efficacy of the Immunogenic Composition Against Atrophic Rhinitis Comprising the rPMT -Efficacy Test Against *P. multocida***

The efficacy test of this Example was conducted based on Taiwan's national regulatory requirement for vaccine against atrophic rhinitis. Thirty-two (32) three-week-old Balb/c mice (The National Laboratory Animal Center, Taiwan) were randomly divided into 3 groups: Group 1 (control, n = 9), Group 2 (rPMT group, n = 12), and Group 3 (Bayovac® group, n = 11). ELISA test showed that all the 32 mice were negative for anti-PMT antibodies before the test was conducted. Each mouse was injected intraperitoneally (i.p.) with 0.5 ml of a ten-fold dilution of the following substance, respectively:

Group 1: PBS solution with 30% (v/v) of aluminum hydroxide gel (negative control);
Group 2: the immunogenic composition against atrophic rhinitis comprising the rPMT (SEQ ID NO: 10) obtained in Example 3 (B.b + PmA+ PmD + rPMT group); and
Group 3: commercial atrophic rhinitis vaccine (Bayovac®, Bayer Taiwan Company Ltd., Taiwan)(Bayovac® group).

Mice of Group 2 and Group 3 were each divided into 3 subgroups on the fourteenth day after immunization. Mice in each subgroup were challenged with $1 \times 10^3$ CFU/ml, $1 \times 10^4$ CFU/ml, $1 \times 10^5$ CFU/ml of a virulent PmD with toxigenicity [obtained from the Animal Health Research Institute, Council of Agriculture, Executive Yuan (Taiwan) as described in Example 2] by intraperitoneal injection, respectively. Mice were observed for 10 days, and then 50% lethal dose ($LD_{50}$) of each group was calculated by Behrens-Karber method with the following formula, in which each immunization group must have a defense index $1 \times 10^{0.5}$ higer than that of a control group.

$$LD_{50} = \text{Minimum dilution fold of challenging dose} - [(\text{sum of mortality rate of each group}/100) - 0.5] \times 1]$$

$$\text{Defense Index} = \{\text{Minimum dilution fold of challenging dose of control group} - [(\text{sum of mortality rate of each group}/100) - 0.5] \times 1\} - \{\text{Minimum dilution fold of challenging dose of immunization group} - [(\text{sum of mortality rate of each group}/100) - 0.5] \times 1\}$$

The results indicate that the immunogenic composition against atrophic rhinitis comprising the rPMT (Group 2, B.b + PmA + PmD + rPMT group) induced a protective response against challenge with a virulent PmD in mice, with a defense index of $10^{2.06}$ (Table 2), which is much higher than that of Taiwan's national regulatory requirement ($10^{0.5}$) and that of commercial atrophic rhinitis vaccine (Bayovac® group)($10^{1.74}$).

Table 2 Results of Immunogenicity and Protection Efficacy of the Immunogenic Composition Against Atrophic Rhinitis Comprising the rPMT

| Group | Challenge concentration | Dilution fold | Mortality | Survival number | Cumulative mortality | Cumulative survival No. | Mortality rate% | LD$_{50}$ | Defense Index |
|---|---|---|---|---|---|---|---|---|---|
| Group 1 (Control group) | $10^4$ | $10^{-5}$ | 2 | 2 | 4 | 2 | 4/6 = 66.7 | $10^{-5.56}$ | --- |
| | $10^3$ | $10^{-6}$ | 1 | 3 | 2 | 5 | 2/7 = 28.6 | | |
| | $10^2$ | $10^{-7}$ | 1 | 3 | 1 | 8 | 1/9 = 11.1 | | |
| Group 2 (B.b + PmA + PmD + rPMT group) | $10^5$ | $10^{-4}$ | 0 | 4 | 0 | 4 | 0/4 = 0 | $10^{-3.5}$ | $10^{2.06}$ |
| | $10^4$ | $10^{-5}$ | 0 | 4 | 0 | 8 | 0/8 = 0 | | |
| | $10^3$ | $10^{-6}$ | 0 | 4 | 0 | 12 | 0/12= 0 | | |
| Group 3 (Bayovac ® group) | $10^5$ | $10^{-4}$ | 0 | 4 | 1 | 4 | 1/5=20 | $10^{-3.82}$ | $10^{1.74}$ |
| | $10^4$ | $10^{-5}$ | 1 | 3 | 1 | 7 | 1/8=12.5 | | |
| | $10^3$ | $10^{-6}$ | 0 | 4 | 0 | 11 | 0/11=0 | | |

**Example 6 Immunogenicity and Protection Efficacy of the Immunogenic Composition Against Atrophic Rhinitis Comprising the rPMT 2**

Sixty-five (65) Balb/c mice (The National Laboratory Animal Center, Taiwan) with a body weight of 15 to 20 g were randomly divided into 3 groups: one control group (Group 1, n = 5) and two immunization groups (Group 2 and Group 3, n = 30 for each group). Each of the two immunization groups was further divided into 3 subgroups of 10 mice. ELISA test showed that all the mice were negative for anti-*P. multocida* antibodies before the test was conducted. Each mouse was injected intraperitoneally (i.p.) with the following substance, respectively:

Group 1: each mouse was injected with 0.2 ml PBS solution (negative control);
Group 2-1: each mouse was injected with 0.2 ml of the stock immunogenic composition against atrophic rhinitis comprising the rPMT (SEQ ID NO: 10) obtained in Example 3 (B.b + PmA + PmD + rPMT group);
Group 2-2: each mouse was injected with 0.2 ml of a 5-fold dilution of the immunogenic composition against atrophic rhinitis comprising the rPMT (SEQ ID NO: 10) obtained in Example 3 (1/5 B.b + PmA + PmD + rPMT group);
Group 2-3: each mouse was injected with 0.2 ml of a 25-fold dilution of the immunogenic composition against atrophic rhinitis comprising the rPMT (SEQ ID NO: 10) obtained in Example 3 (1/25 B.b + PmA + PmD + rPMT group);
Group 3-1: each mouse was injected with 0.2 ml of the stock commercial atrophic rhinitis vaccine (Bayovac®, Bayer Taiwan Company Ltd., Taiwan)(Bayovac® group);
Group 3-2: each mouse was injected with 0.2 ml of a 5-fold dilution of commercial atrophic rhinitis vaccine (Bayovac®, Bayer Taiwan Company Ltd., Taiwan)(1/5 Bayovac® group); and
Group 3-3: each mouse was injected with 0.2 ml of a 25-fold dilution of commercial atrophic rhinitis vaccine (Bayovac®, Bayer Taiwan Company Ltd., Taiwan)(1/25 Bayovac® group).

Fourteen (14) days after the primary immunization (p.i.), the mice of each subgroup were boosted with the same dose of the vaccine, and the mice of control group (Group 1) were injected with 0.2 ml PBS solution again. Ten (10) days after the booster immunization, each mouse was challenged with 0.2 ml of a 100 $LD_{50}$ of a virulent PmD with toxigenicity [obtained from the Animal Health Research Institute, Council of Agriculture, Executive Yuan (Taiwan) as described in Example 2] by intraperitoneal injection. Mice were observed for 10 days, and mortality of each group was recorded. In order to meet the requirement of this Example, the survival rate of each stock immunization subgroup (Group 2-1 and Group 3-1) must be at least 80%, the survival rate of each 5-fold dilution immunization subgroup (Group 2-2 and Group 3-2) must be at least 50%, the survival rate of each 25-fold dilution immunization subgroup (Group 2-3 and Group 3-3) must be at least 20%, and the survival rate of the control group must be 0%.

The results indicate that the immunogenic composition against atrophic rhinitis comprising the rPMT (Groups 2-1, 2-2, and 2-3) induced a sufficiently protective response against challenge with a virulent PmD in mice. All the subgroups of Group 2 meet the requirement, in which the survival rates of stock immunization subgroup (Group 2-1), 5-fold dilution immunization subgroup (Group 2-2), and 25-fold dilution immunization subgroup (Group 2-3) are 90%, 50%, and 30%, respectively.

Table 3 Results of Immunogenicity and Protection Efficacy of the Immunogenic Composition Against Atrophic Rhinitis Comprising the rPMT

| Group | Day after challenging | Mortality | Survival number | Cumulative mortality | Final survival number | Survival rate | Efficacy requirement |
|---|---|---|---|---|---|---|---|
| Group 1 (Control) | 1 | 2 | 3 | 2 | 0 | 0% | 0% (Passed) |
| | 2 | 3 | 0 | 5 | | | |
| Group 2-1 (B.b+PmA+ PmD+rPMT group) | 1 | 0 | 10 | 0 | 9 | 90% | ≥ 80% (Passed) |
| | 2 | 1 | 9 | 1 | | | |
| Group 2-2 (1/5 B.b+PmA+ PmD+rPMT group) | 1 | 2 | 8 | 2 | 5 | 50% | ≥ 50% (Passed) |
| | 2 | 3 | 5 | 5 | | | |
| Group 2-3 (1/25 | 1 | 3 | 7 | 3 | 3 | 30% | ≥ 20% |

| Group | Day after challenging | Mortality | Survival number | Cumulative mortality | Final survival number | Survival rate | Efficacy requirement |
|---|---|---|---|---|---|---|---|
| B.b+PmA+PmD+rPMT group) | 2 | 3 | 4 | 6 | | | (Passed) |
| | 3 | 1 | 3 | 7 | | | |
| Group 3-1 (Bayovac® group) | 1 | 0 | 10 | 0 | 8 | 80% | ≥ 80% |
| | 2 | 2 | 8 | 2 | | | (Passed) |
| Group 3-2 (1/5 Bayovac® group) | 1 | 2 | 8 | 2 | 6 | 60% | ≥ 50% |
| | 2 | 2 | 6 | 4 | | | (Passed) |
| Group 3-3 (1/25 Bayovac® group) | 1 | 2 | 8 | 2 | 2 | 20% | ≥ 20% |
| | 2 | 4 | 4 | 6 | | | (Passed) |
| | 3 | 2 | 2 | 8 | | | |

**Example 7 Immunogenicity and Protection Efficacy of the Immunogenic Composition Against Atrophic Rhinitis Comprising the rPMT - PMT Challenging Test on Mice**

1. Preparation of crude extracted PMT

A virulent PmD with toxigenicity [obtained from the Animal Health Research Institute, Council of Agriculture, Executive Yuan (Taiwan) as described in Example 2] was cultured in Brain Heart Infusion (BHI) broth (BD Biosciences, USA) overnight at 37°C. After that, 100 $\mu$l bacterial culture was evenly plated on 7% blood agar medium and cultured overnight at 37°C, and the bacteria was then collected with PBS solution. The collected bacteria were broken by sonication (SONOPULS, Bandelin, Germany), and toxin was collected by centrifugation (KUBOTA, Japan) followed by filtration of the supernatant with a 0.45 $\mu$m filter (Millipore, USA). The filtered crude extracted PMT was stored at -20°C for further use.

2. Median lethal dose (LD$_{50}$) of crude extracted PMT on mice

Seventy-six (76) three-week-old healthy Balb/c mice (The National Laboratory Animal Center, Taiwan) were randomly divided into 5 groups. ELISA test showed that all the mice were negative for anti *P. multocida* antibodies before the test was conducted. Each mouse was injected intraperitoneally (i.p.) with 0.5 ml crude extracted PMT with different concentration, respectively:

Group 1: 1x10$^8$ CFU/ml crude extracted PMT (n = 14);
Group 2: 2x10$^8$ CFU/ml crude extracted PMT (n = 8);
Group 3: 4x10$^8$ CFU/ml crude extracted PMT (n = 12);
Group 4: 6x10$^8$ CFU/ml crude extracted PMT (n = 20); and
Group 5: 8x10$^8$ CFU/ml crude extracted PMT (n = 22).

Mice were then observed for 10 days, and 50% lethal dose (LD$_{50}$) of the crude extracted PMT was calculated by Calcusyn Software (Biosoft, UK).
The results indicate that median lethal dose (LD$_{50}$) of the crude extracted PMT on mice is 2x10$^8$ CFU/ml (Table 4).

Table 4 Results of median lethal dose (LD$_{50}$) of crude extracted PMT on mice

| PMT Challenged Group | Concentration of Crude Extracted PMT (cfu/ml) | Mortality | Survival number | Cumulative mortality | Final survival number | Mortality rate | Survival rate |
|---|---|---|---|---|---|---|---|
| 1 | $1\times10^8$ | 0 | 10 | 0 | 14 | 0/14 = 0 | 100% |
| 2 | $2\times10^8$ | 4 | 1 | 4 | 4 | 4/8 = 50 | 50% |
| 3 | $4\times10^8$ | 5 | 0 | 9 | 3 | 9/12 = 75 | 25% |
| 4 | $6\times10^8$ | 8 | 2 | 17 | 3 | 17/20 = 85 | 15% |
| 5 | $8\times10^8$ | 4 | 1 | 21 | 1 | 21/22 = 95 | 4.5% |

3. Crude Extracted PMT Challenge on Vaccinated Mice

Twenty-four (24) three-week-old healthy Balb/c mice (The National Laboratory Animal Center, Taiwan) were randomly divided into 5 groups: one control group (Group 1) and four immunization groups (Groups 2 to 5). ELISA test showed that all the 24 mice were negative for anti-PMT antibodies before the test was conducted. Each mouse was injected intraperitoneally (i.p.) with 0.2 ml of the following substance, respectively:

Group 1: PBS solution with 30% (v/v) of aluminum hydroxide gel (negative control);
Group 2: immunogenic composition containing *B. bronchiseptica* (1x10$^9$ CFU/ml), *P. multocida* type A (PmA)(1x10$^9$ CFU/ml), and *P. multocida* type D (PmD)(1x10$^9$ CFU/ml) obtained in Example 2, and 30% (v/v) of aluminum hydroxide gel as adjuvant (B.b + PmA + PmD group);
Group 3: the immunogenic composition against atrophic rhinitis comprising the rPMT (SEQ ID NO: 10) obtained in Example 3 (B.b + PmA+ PmD + rPMT1 group);
Group 4: the immunogenic composition against atrophic rhinitis comprising the rPMT (SEQ ID NO: 11) obtained in Example 3 (B.b + PmA + PmD + rPMT1 group); and
Group 5: commercial atrophic rhinitis vaccine (Bayovac®, Bayer Taiwan Company Ltd., Taiwan)(Bayovac® group).

Fourteen (14) days after primary immunization, each mouse was boosted with the same dose of the substance, respectively. Ten (10) days after the booster immunization, each mouse was challenged with 0.5 ml of a LD$_{85}$ (6x10$^8$ CFU/ml) of crude extracted PMT by intraperitoneal injection. Mice were observed for 10 days, and mortality of each group was recorded.

The results indicate that the immunogenic composition against atrophic rhinitis comprising the recombinant PMT (rPMT1 and rPMT2)(Groups 3 and 4) induced a sufficiently protective response against challenge with crude extracted PMT in mice. The survival rates of Groups 3 and 4 are both higher than the survival rate of negative control and Group 2 (immunization with only *B. bronchiseptica,* PmA, and PmD).

## Table 5 Results of Crude Extracted PMT Challenge on Vaccinated Mice

| Group | Number of mouse | Day after challenging | Mortality | Survival number | Cumulative mortality | Final survival | Survival rate |
|---|---|---|---|---|---|---|---|
| Group 1 (PBS group) | 3 | 1 | 2 | 1 | 2 | 0 | 0% |
| | | 2 | 1 | 0 | 3 | | |
| Group 2 (PmA+PmD+B.b group) | 5 | 1 | 2 | 3 | 2 | 1 | 20% |
| | | 2 | 2 | 1 | 4 | | |
| Group 3 (PmA+PmD+B.b+rPMT1 group) | 5 | 1 | 0 | 5 | 0 | 3 | 60% |
| | | 2 | 2 | 3 | 2 | | |
| Group 4 | 6 | 1 | 1 | 5 | 1 | 3 | 50% |

| Group | Number of mouse | Day after challenging | Mortality | Survival number | Cumulative mortality | Final survival | Survival rate |
|---|---|---|---|---|---|---|---|
| (PmA+PmD+B.b+rPMT2 group) | | 2 | 2 | 3 | 3 | | |
| Group 5 (Bayovac® group) | 5 | 1 | 1 | 4 | 1 | 3 | 60% |
| | | 2 | 1 | 3 | 2 | | |

## Example 8 Immunogenicity of the Immunogenic Composition Against Atrophic Rhinitis Comprising the rPMT - Vaccination Test of Pigs

### 1. Experimental Pigs

Thirty (30) 3 to 4-week-old healthy piglets were randomly chosen in a pig farm (YiLan, Taiwan).

### 2. Vaccination Program

The experimental piglets were ramdonly divided into 3 groups of 10 piglets each. Each piglet was injected intramuscularly with 2 ml of the following substance on day 0 (primary immmunization) and day 21 (booster immunization):

Group 1: PBS solution with 30% (v/v) of aluminum hydroxide gel (negative control);
Group 2: immunogenic composition containing *B. bronchiseptica* ($1 \times 10^9$ CFU/ml), PmA ($1 \times 10^9$ CFU/ml), and PmD ($1 \times 10^9$ CFU/ml) obtained in Example 2, and 30% (v/v) of aluminum hydroxide gel as adjuvant (B.b + PmA + PmD group); and
Group 3: the immunogenic composition against atrophic rhinitis comprising the rPMT (SEQ ID NO: 11) obtained in Example 3 (B.b + PmA + PmD + rPMT group).

Serum samples were collected prior to primary immunization, prior to booster immunization, and 14 days after booster immunization. All the experimental piglets were weighed, and all the serum samples were tested by ELISA.

3. Enzyme-linked immunosorbent assay (ELISA)

Commerical PMT (Merck, USA) was coated on 96-well ELISA plates as antigen for 16 hours at 4°C. Uncoated antigen was removed, and the ELISA plates were washed 3 times with wash buffer (0.9% NaCl and 0.1% Tween 20) and air-dried. To block the ELISA plates, blocking solution (wash buffer containing 1% BSA) was added to the ELISA plates, and then the ELISA plates were incubated for an hour at room temperature. After that, the ELISA plates were washed with wash buffer. Pig serum samples were diluted with PBS and added to the wells of the ELISA plates, and the plates were incubated for an hour at room temperature. After incubation, the serum samples were removed, and the plates were washed with PBS. Secondary antibody conjugated to HRP (goat anti-mouse antibody conjugated HRP, Gene Tex Inc., USA) was diluted 1000-fold with blocking solution and then added to the wells (100 μl/well). After incubating for an hour at room temperature, the secondary antibody was removed, and the plates were washed with wash buffer. For visualization of results, 100 μl of TMB (KPL Inc., USA) was added to each well, and the plates were incubated for 10 minutes in dark. Optical density at 650nm ($OD_{650}$) was read with an ELISA plate reader (SpectraMax® M2/M2 ELISA Reader, Molecular Devices, LLC., USA).

ELISA results are shown in Figure 3. The immunogenic composition against atrophic rhinitis comprising the rPMT (Group 3, B.b + PmA + PmD + rPMT group) induced significantly higher level of serum antibodies against PMT in pigs than Group 1 (control) and Group 2 (B.b + PmA + PmD group) did (**, $p<0.01$). In addition, there was no significant difference in body weights among these three groups (data not shown).

Therefore, the results indicated that the rPMT of the present invention is able to effectively induce immune responses against PMT in animals, especially in pigs, and has no adverse effects on pig growth rates.

**Example 9 Preparation of Anti-RPMT Antibodies**

1. Anti-rPMT Polyclonal Antibodies

The rPMT obtained in Example 1 was mixed with a suitable adjuvant, such as aluminum hydroxide gel. The mixture was primarily inoculated into animals, such as mice, rats, pigs, goats, and rabbits, and a second immunization may be performed after an appropriate time period (such as 2 to 3 weeks) if necessary. After another appropriate time period (such as 2 to 3 weeks), serum of the inoculated animals was collected as anti-rPMT polyclonal antibodies.

The anti-rPMT polyclonal antibodies can be conjugated to chromogenic reporters or fluorescence if necessary.

The inoculated animals can be further vaccinated to boost antibody titer after primary or second immunization if necessary.

Animals that can be inoculated to produce anti-rPMT polyclonal antibodies include, but not limited to, mice, rats, rabbits, avian (eggs), pigs, goats, cattle, and aqua animals.

1. Anti-RPMT Monoclonal Antibodies

The rPMT obtained in Example 1 was mixed with a suitable adjuvant, such as aluminum hydroxide gel. The mixture was primarily inoculated into animals, such as mice, rats, pigs, goats, and rabbits, and a second immunization may be performed after an appropriate time period (such as 2 to 3 weeks) if necessary. After another appropriate time period (such as 2 to 3 weeks), serum of the inoculated animal (such as mice) was collected to evaluate whether the animal's spleen cells were suitable for producing anti-rPMT monoclonal antibodies. Cell fusion of the suitable spleen cells collected from the inoculated animal and myeloma cells (such as FO cell line and NS cell line) was accomplished using polyethylene glycol (PEG, such as PEG1500). Hybridomas that produce antibodies of appropriate specificity were selected from fused cells and next subcloned to be hybridomas that are suitable to produce anti-rPMT monoclonal antibodies.

The anti-rPMT monoclonal antibodies can be used in test kits, therapy, or food or feed supplement to enhance animals' immunity.

**Claims**

1. A recombinant *Pasteurella multocida* toxin, consisting of an amino acid sequence of SEQ ID NO: 10 or 11.

2. A polynucleotide comprising a nucleotide sequence encoding the recombinant *Pasteurella multocida* toxin of claim 1.

3. An immunogenic composition against atrophic rhinitis, comprising the recombinant *Pasteurella multocida* toxin of claim 1 and a pharmaceutically acceptable vehicle.

4. The immunogenic composition of claim 3, further comprising *Bordetella bronchiseptica, Pasteurella multocida* Type A, and *Pasteurella multocida* Type D.

5. The immunogenic composition of claim 3, further comprising at least one pathogen antigen selected from the group consisting of antigen of porcine circovirus type 2 (PCV2), antigen of swine influenza virus (SIV), antigen of porcine reproductive and respiratory syndrome virus (PRRSV), antigen of mycoplasma, antigen of porcine parvovirus (PPV), antigen of erysipelas, antigen of *Actinobacillus pleuropneumonia* (App), and antigen of pseudorabies virus.

6. The recombinant *Pasteurella multocida* toxin of claim 1 for use in protecting an animal against atrophic rhinitis.

7. An anti-*Pasteurella multocida* type D toxin polyclonal antibody against the recombinant *Pasteurella multocida* toxin of claim 1.

8. A test kit for atrophic rhinitis, comprising a detecting unit which is at least one of the recombinant *Pasteurella multocida* toxin of claim 1 and an anti-*Pasteurella multocida* type D polyclonal antibody derived from the recombinant *Pasteurella multocida* toxin of claim 1.

9. The test kit of claim 8, wherein the recombinant *Pasteurella multocida* toxin is deposited on a plate.

**Patentansprüche**

1. Rekombinantes *Pasteurella multocida-Toxin,* bestehend aus einer Aminosäuresequenz der SEQ ID Nr. 10 oder 11.

2. Polynukleotid, umfassend eine Nukleotidsequenz, welche das rekombinante *Pasteurella multocida-Toxin* gemäß Anspruch 1 kodiert.

3. Immunogene Zusammensetzung gegen atrophische Rhinitis, umfassend das rekombinante *Pasteurella multocida-Toxin* gemäß Anspruch 1 und ein pharmazeutisch akzeptables Vehikel.

4. Immunogene Zusammensetzung gemäß Anspruch 3, ferner umfassend, *Bordetella bronchiseptica, Pasteurella multocida* Type A, und *Pasteurella multocida* Type D.

5. Immunogene Zusammensetzung gemäß Anspruch 3, ferner umfassend, mindestens ein pathogenes Antigen ausgewählt aus der Gruppe bestehend aus Antigen des porcinen Zirkovirus Typ 2 (PCV2), Antigen des Schweineinfluenzavirus (SIV), Antigen des porcinen reproduktives und respiratorisches Syndrom Virus (PRRSV), Antigen von Mykoplasma, Antigen des porcinen Parvovirus (PPV), Antigen von Erysipel, Antigen von *Actinobacillus pleuropneumonia* (App) und Antigen des Pseudowut-Virus.

6. Rekombinantes *Pasteurella multocida-Toxin* gemäß Anspruch 1 zur Verwendung beim Schutz eines Tieres gegen atrophische Rhinitis.

7. Poly-klonaler Anti-*Pasteurella-Multocida*-Typ-D-Toxin-Antikörper gegen das rekombinante *Pasteurella multocida-Toxin* gemäß Anspruch 1.

8. Test-Kit für atrophische Rhinitis, umfassend eine Detektionseinheit, welche mindestens eine der folgenden ist, rekombinantes *Pasteurella multocida-Toxin* gemäß Anspruch 1 und ein poly-klonaler Anti-*Pasteurella multocida*-Typ-D-Antikörper, welcher aus dem rekombinanten *Pasteurella multocida-Toxin* gemäß Anspruch 1 erhalten wurde.

9. Test-Kit gemäß Anspruch 8, wobei das rekombinante *Pasteurella multocida*-Toxin auf einer Platte abgelagert ist.

**Revendications**

1. Recombiné de la toxine de *Pasteurella multocida,* constitué par une séquence d'acides aminés de SEQ ID NO: 10 ou 11.

2. Polynucléotide comprenant une séquence de nucléotides codant le recombiné de la toxine de *Pasteurella multocida* selon la revendication 1.

3. Composition immunogène contre la rhinite atrophique, comprenant le recombiné de la toxine de *Pasteurella multocida* selon la revendication 1 et un vecteur pharmaceutiquement acceptable.

4. Composition immunogène selon la revendication 3, comprenant en outre *Bordetella bronchiseptica, Pasteurella multocida* de type A et *Pasteurella multocida* de type D.

5. Composition immunogène selon la revendication 3, comprenant en outre au moins un antigène pathogène qui est sélectionné parmi le groupe qui est constitué par l'antigène du circovirus porcin de type 2 (PCV2), l'antigène du virus de la grippe porcine (SIV), l'antigène du virus du syndrome dysgénésique respiratoire porcin (PRRSV), l'antigène du mycoplasme, l'antigène du parvovirus porcin (PPV), l'antigène de l'érysipèle, l'antigène de l'*Actinobacillus pleuropneumonia* (App) et l'antigène du virus de la pseudorage.

6. Recombiné de la toxine de *Pasteurella multocida* selon la revendication 1 pour une utilisation au niveau de la protection d'un animal contre la rhinite atrophique.

7. Anticorps polyclonal anti-toxine de *Pasteurella multocida* de type D contre le recombiné de la toxine de *Pasteurella multocida* selon la revendication 1.

8. Kit de test pour la rhinite atrophique, comprenant une unité de détection qui est au moins soit le recombiné de la toxine de *Pasteurella multocida* selon la revendication 1, soit un anticorps polyclonal anti-toxine de *Pasteurella multocida* de type D qui est dérivé du recombiné de la toxine de *Pasteurella multocida* selon la revendication 1.

9. Kit de test selon la revendication 8, dans lequel le recombiné de la toxine de *Pasteurella multocida* est déposé sur une lamelle.

Figure 1

Figure 2A                Figure 2B                Figure 2C

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201310095061

- CN 2014073851 W

**Non-patent literature cited in the description**

- **LIAO, C. M. ; HUANG, C. ; HSUAN, S. L. ; CHEN, Z. W. ; LEE, W. C. ; LIU, C. I. ; WINTON, J. R. ; CHIEN, M. S.** Immunogenicity and efficacy of three recombinant subunit Pasteurella multocida toxin vaccines against progressive atrophic rhinitis in pigs. *Vaccine,* 2006, vol. 24, 27-35

- **LEE, J. ; KANG, H. E. ; WOO, H. J.** Protective immunity conferred by the C-terminal fragment of recombinant Pasteurella multocida toxin. *Clin Vaccine Immunol.,* 2012, vol. 19, 1526-1531